# EUROPÄISCHE PATENTANMELDUNG

(11) **EP 2 676 642 A1**
(43) Veröffentlichungstag der Anmeldung: **25.12.2013**
(21) Anmeldenummer: 13165723.1
(22) Anmeldetag: 29.04.2013
(51) Int. Cl.: A61F 2/966

(54) **Freigabevorrichtung zum Lösen eines medizinischen Implantats von einem Katheter**

(30) Priorität: 18.06.2012 US 201261660829 P
(71) Anmelder: Biotronik AG, 8180 Bülach (CH)
(72) Erfinder: Fargahi, Amir, 8180 Bülach (CH)
(74) Vertreter: Lindner-Vogt, Karin L.

(57) **Zusammenfassung**

Die Erfindung betrifft eine Freigabevorrichtung (100, 100e) zum Lösen eines medizinischen Implantats (102) von einer Einführvorrichtung (130, 130e), bei welcher das Implantat (102) durch eine Relativbewegung zwischen einem ersten und einem zweiten Einführelement (52, 52e; 54, 54e) in eine Einführrichtung (56) zuerst an seinem distalen oder proximalen Ende freisetzbar ist. Die Freigabevorrichtung umfasst einen Haltekörper (10, 10a-10e) zum Halten des Implantats (102) in der Einführvorrichtung (130, 130e) mit einem proximalen (12) und einem distalen Ende (14). Der Haltekörper (10, 10a-10e) umfasst einen Anteil (16, 16a-16e; 18, 18a-18c, 18e), der einen Raum (20) zwischen einem der Einführelemente (52, 52e) und dem Anteil des Haltekörpers radial begrenzt. Das Implantat (102) weist zumindest ein Halteelement (104) an zumindest seinem proximalen Ende (106) oder seinem distalen Ende (122) zur Interaktion mit dem Haltekörper (10, 10a-10e) auf.

## Beschreibung

Die Erfindung betrifft eine Freigabevorrichtung zum Lösen eines medizinischen Implantats von einem Katheter und einen Katheter mit einer Freigabevorrichtung zum Freigeben eines medizinischen Implantats zur Implantation in einen tierischen und/oder menschlichen Körper sowie ein Implantat zum Verbinden mit einer solchen Freigabevorrichtung und ein Verfahren zum Halten des Implantats in einer solchen Freigabevorrichtung nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents.

Implantate werden vor einem Einführen in den Körper mit Kathetern verbunden und müssen so befestigt sein, dass sie am Einsatzort komplikationslos vom Katheter genau platziert und definiert freigegeben werden können. Hierzu ist beispielsweise bekannt, das Implantat mit Ösen auszustatten, die mit Haken am Katheter interagieren und so das Implantat am Katheter befestigen.

Aus EP 2 198 805 A1 ist beispielsweise bekannt, an einer Einführvorrichtung und an einem Implantat korrespondierende Befestigungsmittel vorzusehen. Hierbei weist das Implantat beispielsweise radial zur Innenachse weisende Fortsätze auf. In US 8,016,869 B2 ist beispielsweise beschrieben ein proximales Ende eines Implantats mittels Einschieben in eine distale Öffnung eines Befestigungselements an einer Einführvorrichtung zu fixieren.

Der Erfindung liegt die Aufgabe zugrunde, eine Freigabevorrichtung anzugeben, mit der ein Verbinden eines Implantats mit einer Einführvorrichtung einfach und bedienerfreundlich möglich ist und mit der ein hochpräzises und gezieltes Freigeben eines Implantats erfolgen kann.

Eine weitere Aufgabe ist in der Bereitstellung einer entsprechenden Einführvorrichtung zu sehen.

Zudem ergibt sich eine weitere Aufgabe in der Bereitstellung eines Implantats zum Verbinden mit einer solchen Freigabevorrichtung.

Ferner besteht eine weitere Aufgabe darin, ein Verfahren zum Halten des Implantats in einer solchen Freigabevorrichtung bereit zu stellen.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Freigabevorrichtung zum Lösen eines medizinischen Implantats von einer Einführvorrichtung vorgeschlagen, bei welcher das Implantat durch eine Relativbewegung zwischen einem ersten und einem zweiten Einführelement in eine Einführrichtung zuerst an seinem distalen Ende freisetzbar ist. Die Freigabevorrichtung umfasst einen Haltekörper zum Halten des Implantats in der Einführvorrichtung, mit einem proximalen Ende, das im Benutzungszustand von einem distalen Ende der Einführvorrichtung entfernt ist, und einem distalen Ende, das im Benutzungszustand dem distalen Ende der Einführvorrichtung zugewandt ist, wobei der Haltekörper zumindest einen Anteil aufweist, der zumindest einen Raum radial begrenzt, der sich zwischen einem der Einführelemente und dem zumindest einen Anteil erstreckt, wobei das Implantat zumindest ein Halteelement an zumindest seinem proximalen Ende zur Interaktion mit dem Haltekörper aufweist, und wobei zumindest ein Bereich des zumindest einen Halteelements bei der Interaktion mit dem Haltekörper in Einführrichtung in den zumindest einen Raum einführbar und/oder entgegen der Einführrichtung aus dem zumindest einen Raum ausführbar ist.

Alternative wird vorgeschlagen, dass das Implantat zuerst an seinem proximalen Ende freisetzbar ist und zumindest ein Halteelement an zumindest seinem distalen Ende zur Interaktion mit dem Haltekörper aufweist, und wobei zumindest ein Bereich des zumindest einen Halteelements bei der Interaktion mit dem Haltekörper entgegen der Einführrichtung in den zumindest einen Raum einführbar und/oder in Einführrichtung aus dem zumindest einen Raum ausführbar ist.

Durch die erfindungsgemäßen Ausgestaltungen kann jeweils eine Freigabevorrichtung bereitgestellt werden, in der das Implantat sicher gehalten wird. Zudem wird so ein einfaches Konzept zum Halten und zu einer Freigabe des Implantats verwirklicht. Durch den Haltekörper kann eine unerwünschte Bewegung des Implantats, z.B. ein so genanntes Jumping, bei einer Zuführung des Implantats an einen Implantationsort und/oder der Freigabe des Implantats verhindert werden. Der Haltekörper erlaubt ferner eine kompakte und einfache Konstruktion der jeweiligen Freigabevorrichtung. Zudem weist die jeweilige Freigabevorrichtung eine einfache Handhabung und Montage des Implantats auf der entsprechenden Einführvorrichtung, beispielsweise einem Katheter, im Vorbereitungslabor auf. Des Weiteren ist die Freigabe des Implantats zuverlässig und schnell. Ferner kann ein Risiko einer Deformation des Implantats sowie einer daraus resultierende Blockade der jeweiligen Freigabevorrichtung, wie sie bei Vorrichtungen des Standes der Technik, die mit Haken und Ösen arbeiten, auftreten, eliminiert werden. Zudem ist ein solcher Haltekörper besonders patientenfreundlich, da ein Risiko für eine Scherung von Haken und Ösen durch das Implantat und daraus resultierende gelöste Partikel im Körper des Patienten, wie sie bei Vorrichtungen des Standes der Technik auftreten können, verhindert werden. Besonders vorteilhaft kann mittels der erfindungsgemäßen Ausgestaltungen eine Repositionierung des teilweise freigegebenen Implantats ermöglicht werden. Dies ist insbesondere der Fall, da der Haltekörper eine große Haltekraft erzeugen kann. Ferner hat die Repositionierung keine negative Wirkung auf die Interaktion zwischen dem Haltekörper und dem zumindest einen Halteelement des Implantats. Zudem kann ein Funktionstest des Implantats und im Defektfall eine Entfernung des missfunktionalen Implantats erfolgen. Die Freigabe des Implantats gemäß der alternativen Ausgestaltung mit dem proximalen Ende zuerst ist besonders geeignet für asymmetrische Implantate, die beispielsweise bei Herzkatheteranwendungen zum Einsatz kommen.

In diesem Zusammenhang soll unter einem "Haltekörper" ein Körper verstanden werden, der mittels einer Haltewirkung, eines Formschlusses und/oder eines Kraftschlusses ein anderes Element, insbesondere das Implantat, an einem Bauteil der Einführvorrichtung, insbesondere einem Einführelement bzw. dem Innenschaft, in einer festgelegten Position hält. Hierbei kann der Haltekörper die Haltewirkung selbst vermitteln oder er kann mit zumindest einem weiteren Element zusammenwirken. Die kann insbesondere ein Element der Einführvorrichtung, wie beispielsweise einem Einführelement bzw. dem Innenschaft und/oder einem Außenschaft der Einführvorrichtung oder einem Stopper, oder des Implantats sein. Zusätzlich kann die Haltewirkung durch eine Materialeigenschaft des Haltekörpers oder des anderen Elements unterstützt oder bewirkt werden. Unter dem Begriff "bewirken" soll hier und im weiteren Text "erzeugen, veranlassen und/oder erreichen" verstanden werden.

Unter einem Anteil des Haltekörpers soll ein Bereich des Haltekörpers verstanden werden, der mit einem radialen Abstand zu einem Einführelement und insbesondere zu dem inneren Einführelement angeordnet ist. Dadurch ist radial zwischen dem inneren Einführelement und dem zumindest einen Anteil ein Raum angeordnet bzw. erstreckt sich dieser zwischen dem inneren Einführelement und dem zumindest einen Anteil, wobei der zumindest eine Anteil den Raum an zumindest einer Seite radial begrenzt. Der Raum ist vorteilhaft von einem Hohlraum und insbesondere von einem an zumindest einer Seite offenen Hohlraum gebildet. Eine stabile und behinderungsfreie Interaktion zwischen dem zumindest einen Bereich des Halteelements und des Haltekörpers kann erreicht werden, wenn der zumindest eine Raum an dem proximalen Ende oder an dem distalen Ende des Haltekörpers angeordnet ist.

In diesem Zusammenhang soll unter einem Halteelement des Implantats ein Element verstanden werden, das eine Haltewirkung vermittelt insbesondere über einen Formschlusses und/oder einen Kraftschlusses. Zudem ist das Halteelement so ausgebildet, dass es mit dem Haltekörper interagieren kann. Das Halteelement umfasst zumindest eine Richtungsänderung, die relativ zu einem Bereich des Implantats, der sich im Wesentlichen parallel zu einer Innenachse des Implantats erstreckt, einen Gesamtwinkel von mehr als 90°, bevorzugt von mehr als 120° und besonders bevorzugt von mehr als 150° aufweist und in radialer Richtung zu der Innenachse orientiert angeordnet ist. Unter "im Wesentlichen parallel" soll eine Abweichung der Richtung des Bereichs von der Richtung der Innenachse von bis zu 30° zu deren parallelen Anordnung als "parallel" verstanden werden. Ferner definiert sich ein Gesamtwinkel entweder als ein Winkel einer Richtungsänderung oder als ein summierter Winkel von Winkeln von zwei und/oder mehreren Richtungsänderungen. Unter einer Richtungsänderung soll insbesondere eine Richtungsumkehr verstanden werden. Unter der Wendung "in radialer Richtung zu der Innenachse orientiert angeordnet" soll verstanden werden, dass das Halteelement in einem Bereich radial zwischen einer Wand des Implantats und der Innenachse angeordnet ist. Es soll darunter insbesondere nicht verstanden werden, dass der Bereich, der in den Raum des Haltekörpers ein- oder ausführbar ist radial zur Innenachse weist.

Zudem kann das Halteelement von jedem, dem Fachmann für einsetzbar erachteten Element gebildet sein, wie einem gebogenen Stab, einem Stab mit Kugel am radial inneren Ende, einem Zacken oder bevorzugt einem Haken. Das Halteelement kann zur Verstärkung der Haltekraft so ausgeführt sein, beispielsweise durch eine Federvorspannung, dass es im verbundenen Zustand mit dem Haltekörper eine Klemmkraft auf diesen ausübt. Bevorzugt sind zwei und/oder mehrere Halteelemente gleichmäßig über eine Außenfläche bzw. bei einem runden Implantat gleichmäßig über einen Umfang des Implantats verteilt angeordnet. Grundsätzlich wäre auch eine ungleichmäßige Verteilung denkbar.

In diesem Zusammenhang soll unter der Einführrichtung die Richtung verstanden werden, die vom proximalen zum distalen Ende der Einführvorrichtung weist. Insbesondere werden das Implantat und die Freigabevorrichtung in Einführrichtung mit der Einführvorrichtung in den Körper eingeführt. Unter "einführbar" und/oder "ausführbar" soll eine Bewegung in den Raum und/oder aus dem Raum des Haltekörpers verstanden werden. Hierbei kann die Bewegung zum Ein- oder Ausführen durch ein direktes Angreifen, beispielsweise eines Monteurs, an das Implantat oder durch ein indirektes Angreifen an das Implantat, beispielsweise mittels eines Einführelements, wie dem äußeren Einführelement, vermittelt werden. Ferner kann auch der Haltekörper zum Ein- oder Ausführen relativ zum Implantat, beispielsweise über die Bewegung eines Einführelements, wie dem inneren Einführelement, bewegt werden.

Beginnt die Freigabe des Implantats an dessen distalem Ende ist die Freigabevorrichtung einsetzbar für eine so genannte distale Einführvorrichtung. Hierbei ist das innere Einführelement (erstes Einführelement) mit einer Spitze der Einführvorrichtung, wie einer Katheterspitze, verbunden. Das äußere Einführelement (zweites Einführelement), welches radial um das innere Einführelement angeordnet ist, ist hingegen nicht mit der Spitze verbunden und kann durch eine axiale Bewegung in Richtung des proximalen Endes der Einführvorrichtung relativ zu der Spitze bewegt werden. Dadurch kann ein radial zwischen dem inneren und äußeren Einführelement angeordnetes Implantat zu dessen Implantation bzw. Expansion mit seinem distalen Ende zuerst freigelegt werden.

Ist die Freigabevorrichtung gemäß der alternativen Ausführung, bei der die Freigabe des Implantats an dessen proximalen Ende beginnt, gestaltet, ist die Freigabevorrichtung einsetzbar für eine so genannte proximale Einführvorrichtung. Bei dieser ist das äußere Einführelement mit der Spitze der Einführvorrichtung verbunden, das innere Einführelement hingegen nicht. Zu einem Bewegen des äußeren Einführelements ist dieses mit einem Führungselement gekoppelt. Das Führungselement verläuft koaxial zu dem inneren Einführelement und in diesem und ist beispielsweise von einem Schaft mit einem Einführungsdraht und einem Lumen gebildet. Zu einer Manipulation durch einen Bediener ist das Führungselement mit dem proximalen Ende der Einführvorrichtung verbunden. Auch das Führungselement ist mit der Spitze verbunden. Wird nun das Einführelement in Richtung des distalen Endes der Einführvorrichtung verschoben, drückt es sowohl die Spitze wie auch das äußere Einführelement in die distale Richtung, wodurch ein radial zwischen dem inneren und äußeren Einführelement angeordnetes Implantat zu dessen Implantation bzw. Expansion mit seinem proximalen Ende zuerst freigelegt werden kann.

Vorteilhafterweise hält in einem Haltezustand ein Formschuss zwischen dem Anteil des Haltekörpers und des zumindest einen Halteelements des Implantats dieses in Position, wodurch ein Entgleiten des Implantats aus der Freigabevorrichtung bzw. des Haltekörpers verhindert wird. Ein "Haltezustand" stellt hier einen Zustand dar in dem das Implantat verliersicher in der Einführvorrichtung gehalten wird.

Vorteilhafterweise ist der Haltekörper an einem der Einführelemente und insbesondere dem inneren Einführelement befestigt. Hierdurch können der Haltekörper und damit das Implantat bei einer Implantation besonders verliersicher mit dem Einführelement bewegt werden. Der Raum zum Ein- oder Ausführen des Halteelements des Implantats kann komfortabel groß ausgestaltet werden, wenn der Haltekörper mit seinem proximalen Ende oder seinem distalen Ende an einem der Einführelemente und insbesondere dem inneren Einführelement befestigt ist.

In einer alternativen oder zusätzlichen Ausgestaltung kann der Haltekörper an einem anderen Element der Einführvorrichtung und insbesondere an dem auf dem inneren Einführelement angeordneten Stopper befestigt sein. Dies erfolgt beispielsweise über Stege, die am proximalen Ende des Haltekörpers angeordnet und/oder angeformt sind.

Es kann jede, dem Fachmann für sinnvoll erachtete Verbindungsart, wie ein Kraft-, ein Form- oder ein Stoffschluss, beispielsweise mittels Schweißen, Löten, Schrauben, Nageln oder Kleben, für beide Befestigungsvarianten in Frage kommen. Eine konstruktiv einfach zu erhaltende Befestigung kann erreicht werden, wenn der Haltekörper auf das innere Einführelement und/oder an den Stopper geklebt ist. Vorteilhafterweise erfolgt dies mittels eines UV-aushärtbaren Klebstoffs, wodurch eine gut kontrollierbare und steuerbare Prozedur angewendet werden kann.

In einer vorteilhaften Realisierung weist der Haltekörper zumindest zwei Anteile auf, die jeweils zumindest einen Raum radial begrenzen, der sich jeweils zwischen einem inneren Einführelement und dem Anteil erstreckt. Bevorzugt weist das Implantat eine entsprechende Anzahl an Halteelementen auf. Somit kann das Implantat besonders homogen und gleichmäßig mit dem Haltekörper verbunden werden. Die Anteile und damit auch die entsprechenden Räume sind bevorzugt gleichmäßig über eine Außenfläche bzw. bei einem runden Haltekörper gleichmäßig über einen Umfang des Haltekörpers verteilt angeordnet bzw. liegen sich bezogen auf das innere Einführelement radial gegenüber. Grundsätzlich wäre auch eine ungleichmäßige Verteilung denkbar. Der Haltekörper kann auch mehr als zwei Anteile bzw. dazugehörende Räume aufweisen.

Eine besonders stabile und zuverlässige Konstruktion des Haltekörpers kann bereitgestellt werden, wenn die zumindest zwei Anteile an demselben Bauteil ausgebildet sind. In anderen Worten, die zumindest zwei Anteile sind einstückig miteinander ausgeführt. Hierbei soll unter "einstückig" verstanden werden, dass ein erster Anteil und ein zweite Anteil nur unter Funktionsverlust zumindest eines der Anteile voneinander getrennt werden können. Das Bauteil kann jede beliebige Querschnittsform, wie eckig, drei- oder viereckig, hantelförmig, oval oder insbesondere rund, aufweisen.

Bevorzugt sind die zumindest zwei Anteile in einer Region eines Bauteils angeordnet, die sich in Umfangsrichtung um eines der Einführelemente, insbesondere dem inneren Einführelement, erstreckt. Durch diese Ausgestaltung kann der Haltekörper vorteilhaft auf eine runde Form des Implantats angepasst sein, was eine exakte Verbindung zwischen dem Haltekörper und den Halteelementen ermöglicht. Das Bauteil mit der runden Querschnittsform kann von einem Rohr, einem Zylinder und besonders vorteilhaft von einer Buchse gebildet sein. Ein Zylindermantel der Buchse erstreckt sich bevorzugt im Wesentlichen parallel zu dem inneren Einführelement. Unter "im Wesentlichen parallel" soll eine Abweichung der Richtung des Anteils von der Richtung des inneren Einführelements von bis zu 30° zu deren parallelen Anordnung als "parallel" verstanden werden. Solch ein Zylindermantel ist kommerziell verfügbar, wodurch auf eine aufwendige Konstruktion eines entsprechenden Bauteils Zeit und Kosten sparend verzichtet werden kann. Ein sich radial um das Einführelement erstreckender Boden der Buchse wird bevorzugt von einer Klebstoffschicht aus dem Klebstoff zur Befestigung des Haltekörpers auf dem Einführelement gebildet.

In einer alternativen Ausgestaltung sind die zumindest zwei Anteile an zwei unterschiedlichen Bauteilen ausgebildet und/oder von zwei unterschiedlichen Bauteilen gebildet, wodurch jedes individuell auf unterschiedliche Gegebenheiten des Implantats, wie Länge, Krümmung etc., oder voneinander anweichende Funktionen, wie die leichtere und damit schnellere Freigabe des Halteelements oder dergleichen, abgestimmt werden können. Die obigen Ausführungen zu den einstückig ausgeführten Anteilen gelten auch für die hier beschriebenen Anteile.

In einer bevorzugten Realisierung weist der Haltekörper ein Material auf, das ausgewählt ist aus der Gruppe bestehend aus einer Keramik, einem Glas, einem Gummi, einem Kunststoff, einem Metall und einem röntgensichtbarem Metall, wodurch eine Vielzahl leicht zugänglicher und in ihren Eigenschaften bekannte Materialien zum Einsatz kommen können. Vorteilhaft ist das Material ein Kunststoff wie Polyamid (PA) oder AcrylnitrilButadien-Styrol (ABS). Ein solches Material ermöglicht eine leichte Herstellung des Haltekörpers, da sie sich gut spritzen lassen. Damit sind sie ideal für Bauteile mit dünnen Wandstärken. Ferner sind diese Materialien biokompatibel. Da diese Materialien bruchfest sind, kann ein Haltekörper aus einem dieser Materialien zudem besonders widerstandsfähig realisiert werden.

Alternativ ist das Material ein Metall, wie Stahl, Nitinol, Tantal, Gold oder Platin. Dadurch kann ein insbesondere gegen Körperflüssigkeiten resistentes Material eingesetzt werden. Bevorzugt ist das Metall ein röntgensichtbares Metall, wie Tantal, Gold oder Platin, wodurch der Haltekörper Platz und Montageaufwand sparend eine weitere Funktion aufweist.

Zu einer Erhöhung der Haltekraft kann eine Fläche des zumindest einen Anteils des Haltekörpers, die das zumindest eine Halteelement des Implantats kontaktiert, und/oder eine Fläche des zumindest einen Halteelements des Implantats, die den zumindest einen Anteil des Haltekörpers kontaktiert, eine Beschichtung aufweisen, die zur Erhöhung der Reibung zwischen diesen Bauteilen dient. Hierfür weist die Beschichtung bevorzugt ein Material mit hoher Haftreibung auf, um das Implantat im Haltezustand in Position zu halten, wodurch eine Fixierung des Implantats konstruktiv einfach erfolgen kann. Das Material kann jedes, vom Fachmann für sinnvoll erachtetes Material sein, wie beispielsweise ein Polymer und insbesondere ein Material ausgewählt aus der Gruppe bestehend aus Polyamid, Polyester, Polyether-Block-Amid, Silikon, Polyurethan und besonders bevorzugt ein Polyether-Block-Amid, wie PEBAX der Firma Arkema. Zu einer guten Kontaktierung der jeweiligen Fläche und der Beschichtung kann eine weitere Schicht bzw. eine Verbindungsschicht vorgesehen sein, die bevorzugt aus einem Material gefertigt ist, das als ein Haftvermittler geeignet ist, wie bspw. das Material linear low density Polyethylen (LLDP).

In einer weiteren Ausgestaltung der Erfindung wird vorgeschlagen, dass der Haltekörper eine Durchführung für eines der Einführelemente aufweist. Dies erlaubt eine kompakte Anordnung, welche das durchgeführte Einführelement stabilisiert und schützt. Ist die Einführvorrichtung ein Katheter, so kann das betreffende Einführelement ein Innenschaft des Katheters sein.

Gemäß einem weiteren Aspekt der Erfindung wird eine Einführvorrichtung zum Einführen des medizinischen Implantats vorgeschlagen, welches durch eine Relativbewegung zwischen dem ersten und dem zweiten Einführelement in Einführrichtung zuerst an seinem distalen Ende freisetzbar ist, umfassend die Freigabevorrichtung zum Lösen des medizinisches Implantats, umfassend den Haltekörper zum Halten des Implantats in der Einführvorrichtung, mit dem proximalen Ende, das im Benutzungszustand von dem distalen Ende der Einführvorrichtung entfernt ist, und dem distalen Ende, das im Benutzungszustand dem distalen Ende der Einführvorrichtung zugewandt ist, wobei der Haltekörper zumindest einen Anteil aufweist, der zumindest einen Raum radial begrenzt, der sich zwischen einem der Einführelemente und dem zumindest einen Anteil erstreckt, wobei das Implantat das zumindest eine Halteelement an zumindest seinem proximalen Ende zur Interaktion mit dem Haltekörper aufweist, und wobei der zumindest eine Bereich des zumindest einen Halteelements bei der Interaktion mit dem Haltekörper in Einführrichtung in den zumindest einen Raum einführbar und/oder entgegen der Einführrichtung aus dem zumindest einen Raum ausführbar ist.

In einer alternativen Ausgestaltung ist das Implantat zuerst an seinem proximalen Ende freisetzbar und weist zumindest ein Halteelement an zumindest seinem distalen Ende zur Interaktion mit dem Haltekörper auf, und wobei zumindest ein Bereich des zumindest einen Halteelements bei der Interaktion mit dem Haltekörper entgegen der Einführrichtung in den zumindest einen Raum einführbar und/oder in Einführrichtung aus dem zumindest einen Raum ausführbar ist.

Durch die erfindungsgemäßen Ausgestaltungen kann jeweils eine Einführvorrichtung bereitgestellt werden, in der das Implantat sicher gehalten wird. Ferner kann eine unerwünschte Bewegung des Implantats, z.B. Jumping, bei der Zuführung des Implantats an den Implantationsort und/oder der Freigabe des Implantats verhindert werden. Die jeweilige Freigabevorrichtung weist somit eine kompakte und einfache Konstruktion auf. Zudem kann die Handhabung und Montage der entsprechenden Einführvorrichtung, beispielsweise einem Katheter, im Vorbereitungslabor einfach gestaltet werden. Die Freigabe des Implantats ist vorteilhaft zuverlässig und schnell. Ferner kann die Blockade der jeweiligen Freigabevorrichtung durch das minimierte Risiko der Deformation des Implantats eliminiert werden. Besonders vorteilhaft kann mittels der erfindungsgemäßen Ausgestaltung die Repositionierung des teilweise freigegebenen Implantats ermöglicht werden. Zudem kann ein Funktionstest des Implantats und im Defektfall die Entfernung des missfunktionalen Implantats erfolgen. Die Einführvorrichtung gemäß der alternativen Ausgestaltung mit der Freigabe des proximalen Endes des Implantats zuerst kann besonders vorteilhaft bei Implantationen von asymmetrischen Implantaten, wie beispielsweise bei Herzkatheteranwendungen, eingesetzt werden.

Zudem wird vorgeschlagen, dass die Einführvorrichtung einen Stopper aufweist, der eine Bewegung des Implantats in Richtung des proximalen Endes der Einführvorrichtung oder in Richtung des distalen Endes der Einführvorrichtung limitiert. Solch eine Bewegung würde unvorteilhaft zu einer Blockade des Implantates führen. Eine bevorzugte Weiterbildung besteht darin, dass der Stopper ein Ausstoßen des Implantats bewirkt, wodurch das Freisetzen des Implantats unkompliziert und ohne zusätzliche Aktion eines Bedieners der Freigabevorrichtung, die eine Implantation des Implantats unnötig verlängert, erfolgt. Bezüglich der Begriffsdefinition zu dem Begriff "bewirken" wird auf die Ausführungen oben verwiesen. Der Stopper kann aus jedem der oben genannten Materialien gefertigt sein. Bevorzugt weist der Stopper als Material ein Polymer auf und insbesondere ein Polymer ausgewählt aus der Gruppe bestehend aus Polyester, Polyether-Block-Amid, Silikon, Polyurethan und Polyamid. Bevorzugterweise besteht der Stopper aus Polyamid.

Die Erfindung geht ferner aus von einem medizinischen Implantat mit einem Grundkörper zum Verbinden mit der Freigabevorrichtung und zum Lösen von der Einführvorrichtung, wobei das Implantat durch eine Relativbewegung zwischen dem ersten und dem zweiten Einführelement in Einführrichtung zuerst an seinem distalen Ende freisetzbar ist, aufweisend zumindest ein Halteelement zur Interaktion mit dem Haltekörper der Freigabevorrichtung, wobei das zumindest eine Halteelement zumindest eine Richtungsänderung umfasst, die relativ zu einem Bereich des Grundkörpers, der sich im Wesentlichen parallel zu einer Innenachse des Grundkörpers erstreckt, einen Gesamtwinkel von mehr als 90° aufweist und in radialer Richtung zu der Innenachse orientiert angeordnet ist, so dass bei der Interaktion mit dem Haltekörper der zumindest eine Bereich des zumindest einen Halteelements in Einführrichtung in den zumindest einen Raum des Haltekörpers einführbar und/oder entgegen der Einführrichtung aus dem zumindest einen Raum ausführbar ist.

Alternativ ist das Implantat zuerst an seinem proximalen Ende freisetzbar und es ist bei der Interaktion mit dem Haltekörper zumindest ein Bereich des zumindest einen Halteelements entgegen der Einführrichtung in den zumindest einen Raum des Haltekörpers einführbar und/oder in Einführrichtung aus dem zumindest einen Raum ausführbar.

Durch die erfindungsgemäßen Ausgestaltungen kann ein Implantat bereitgestellt werden, das an der jeweiligen Freigabevorrichtung sicher gehalten werden kann. Zudem wird so ein einfaches Konzept zum Halten und zu einer Freigabe des Implantats verwirklicht. Durch das zumindest eine Halteelement kann eine unerwünschte Bewegung des Implantats, z.B. Jumping, bei der Zuführung des Implantats an den Implantationsort und/oder der Freigabe des Implantats verhindert werden. Des Weiteren ist die Freigabe des Implantats zuverlässig und schnell. Ferner kann das Risiko einer Deformation des Implantats sowie der daraus resultierende Blockade der entsprechenden Freigabevorrichtung, wie sie bei Vorrichtungen des Standes der Technik, die mit Haken und Ösen arbeiten, auftreten, eliminiert werden. Besonders vorteilhaft kann mittels der erfindungsgemäßen Ausgestaltungen die Repositionierung des teilweise freigegebenen Implantats ermöglicht werden. Zudem kann ein Funktionstest des Implantats und im Defektfall die Entfernung des missfunktionalen Implantats erfolgen. Die Ausgestaltung bzw. Freisetzung des Implantats gemäß der alternativen Ausgestaltung mit dem proximalen Ende zuerst ist besonders geeignet für asymmetrische Implantate, die beispielsweise bei Herzkatheteranwendungen zum Einsatz kommen.

Das zumindest eine Halteelement ist bevorzugt an zumindest dem distalen Ende oder dem proximalen Ende des Implantats angeordnet und/oder angeformt. Ferner soll in diesem Zusammenhang unter einem "Grundkörper" insbesondere eine Struktur, wie beispielsweise ein Drahtgeflecht, verstanden werden, die im Wesentlichen eine Gestalt und/oder eine Form des Implantats bzw. das Implantat selbst bildet. Durch diese Struktur weist der Grundkörper zumindest eine Basisstruktur auf. Das zumindest eine Halteelement lässt sich konstruktiv besonders einfach realisieren, wenn es zumindest zu mehr als 50%, bevorzugt zu mehr als 75% und besonders bevorzugt zu mehr als 90% die Basisstruktur des Grundkörpers aufweist. Vorteilhafterweise ist das zumindest eine Halteelement von der Basisstruktur des Grundkörpers gebildet.

Gemäß einer weiteren vorteilhaften Realisierung weist das zumindest eine Halteelement einen Bereich auf, der sich im Wesentlichen in radialer Richtung zu der Innenachse des Implantats erstreckt und in seiner Länge mehr als einmal so lang ist wie eine Materialstärke des Anteils des Haltekörpers der Freigabevorrichtung. Dadurch kann eine Interaktion zwischen dem Haltekörper und dem Halteelement bevorzugt reibungslos erfolgen. Damit ist die Interaktion unabhängig von einer Genauigkeit von Dimensionen des Haltekörpers und des zumindest einen Halteelements und von Toleranzen bei deren Herstellung, wie dies beides bei Vorrichtungen des Standes der Technik der Fall sein muss. Unter "im Wesentlichen in radialer Richtung" soll eine Abweichung der Richtung des Bereichs von der radialen Richtung zu der Innenachse von bis zu 30° zu deren senkechten bzw. radialen Anordnung als "in radialer Richtung" verstanden werden.

Gemäß einer vorteilhaften Ausgestaltung kann das Implantat ein selbstexpandierendes Implantat sein, wodurch es sich bei der Freigabe selbsttätig öffnen kann. Durch das selbstexpandierende Implantat kann ein zusätzliches Expandiermittel entfallen. Dadurch können vorteilhaft Raum und Montageaufwand für dieses eingespart werden. Hierdurch kann auch die Einführvorrichtung weniger komplex gestaltet werden. Grundsätzlich wäre es jedoch auch möglich ein ballonexpandierbares Implantat zu verwenden. Hierfür müsste die Einführvorrichtung jedoch entsprechend angepasst werden, was der Fachmann aufgrund seiner Fachkenntnis selbstständig löst.

Ferner geht die Erfindung aus von einem Verfahren zur Herstellung eines erfindungsgemäßen Implantats mit einem Grundkörper, aufweisend zumindest eine Basisstruktur und zumindest ein Halteelement zur Interaktion mit einem Haltekörper einer erfindungsgemäßen Freigabevorrichtung, wobei das zumindest eine Halteelement zumindest zu mehr als 50%, bevorzugt zu mehr als 75% und besonders bevorzugt zu mehr als 90% die Basisstruktur aufweist, wobei das zumindest eine Halteelement durch Biegen zumindest eines Anteils eines Endes des Implantats, insbesondere des distalen und/oder proximalen Endes, radial in Richtung einer Innenachse des Implantats bzw. Grundkörpers erhalten wird. Durch die erfindungsgemäße Ausgestaltung kann schnell und aufwandsgünstig ein stabiles und kompaktes Implantat hergestellt werden.

Die Erfindung geht zudem aus von einem Verfahren zum Verbinden des medizinischen Implantats mittels des Haltekörpers der Freigabevorrichtung in der Einführvorrichtung, bei welcher das Implantat durch eine Relativbewegung zwischen dem ersten und dem zweiten Einführelement in Einführrichtung zuerst an seinem distalen Ende freigesetzt wird. Hierbei umfasst der Haltekörper das proximale Ende, das im Benutzungszustand vom distalen Ende der Einführvorrichtung entfernt ist, und das distale Ende, das im Benutzungszustand dem distalen Ende der Einführvorrichtung zugewandt ist. Das Verfahren weist zumindest die folgenden Schritte auf: Platzieren zumindest des proximalen Endes des Implantats entgegen der Einführrichtung an einem der Einführelemente, insbesondere dem inneren Einführelement, so dass zumindest das proximale Ende des Implantats proximal von dem Haltekörper angeordnet ist; Bewegen zumindest des proximalen Endes des Implantats in Einführrichtung, so dass zumindest der Bereich des Halteelements des proximalen Endes des Implantats in Einführrichtung in den vom Haltekörper radial begrenzten Raum eingeführt wird; Platzierung des Haltekörpers mit dem Implantat in zumindest einem der Einführelement, insbesondere dem äußeren Einführelement.

Gemäß eines alternativen Verfahrens wird das Implantat zuerst an seinem proximalen Ende freigesetzt und weist diese zumindest die folgenden Schritte auf: Platzieren zumindest eines distalen Endes des Implantats in Einführrichtung an einem der Einführelemente, so dass zumindest das distale Ende des Implantats distal von dem Haltekörper angeordnet ist; Bewegen zumindest des distalen Endes des Implantats entgegen der Einführrichtung, so dass zumindest ein Bereich zumindest eines Halteelements des distalen Endes des Implantats entgegen der Einführrichtung in einen vom Haltekörper radial begrenzten Raum eingeführt wird; Platzierung des Haltekörpers mit dem Implantat in zumindest einem der Einführelemente. Das Verfahren gemäß der alternativen Ausgestaltung bei dem zuerst das proximale Ende des Implantats freigegeben wird, ist besonders geeignet für die Implantation von asymmetrischen Implantaten, wie diese beispielsweise bei Herzkatheteranwendungen zum Einsatz kommen.

Durch die erfindungsgemäßen Ausgestaltungen kann jeweils ein Verfahren realisiert werden, mittels dem eine Verbindung zwischen dem Implantat und der Freigabevorrichtung bedienerfreundlich, zuverlässig und schnell hergestellt werden kann. Bei einem so mit der Freigabevorrichtung verbundenen Implantat kann eine unerwünschte Bewegung des Implantats, z.B. Jumping, bei der Zuführung des Implantats an den Implantationsort und/oder der Freigabe des Implantats verhindert werden. Zudem ist die Handhabung und Montage des Implantats in der Einführvorrichtung, beispielsweise einem Katheter, im Vorbereitungslabor stark vereinfacht. Des Weiteren kann die Freigabe des Implantats zuverlässig und schnell erfolgen. Besonders vorteilhaft kann ein mittels der erfindungsgemäßen Verfahren mit der Freigabevorrichtung verbundenes und schon teilweise freigegebenes Implantat bedienerfreundlich repositioniert werden. Zudem kann an einem so befestigten Implantat ein Funktionstest durchgeführt werden und dieses kann im Defektfall entfernt werden.

Bevorzugt erfolgt das Platzieren zumindest des distalen Endes des Implantats in Einführrichtung oder des proximalen Endes des Implantats entgegen der Einführrichtung an einem der Einführelemente, insbesondere dem inneren Einführelement, bis das distale Ende bzw. das proximale Ende an dem Stopper anschlägt. Das Bewegen zumindest des distalen Endes entgegen der Einführrichtung oder proximalen Endes des Implantats in Einführrichtung erfolgt beispielsweise mittels Bewegung des Außenschlauchs entgegen der Einführrichtung oder in Einführrichtung.

Des Weiteren geht die Erfindung aus von einem Verfahren zum Betreiben der Freigabevorrichtung umfassend den Haltekörper, mit dem proximalen Ende, das im Benutzungszustand von dem distalen Ende der Einführvorrichtung entfernt ist, und dem distalen Ende, das im Benutzungszustand dem distalen Ende der Einführvorrichtung zugewandt ist, und zum Lösen des medizinischen Implantats von der Einführvorrichtung in Einführrichtung, bei welcher das Implantat zwischen dem ersten und dem zweiten Einführelement angeordnet ist, umfassend zumindest die folgenden Schritte: Bewegen zumindest des proximalen Endes des Implantats entgegen der Einführrichtung, so dass zumindest ein Bereich eines Halteelements des proximalen Endes des Implantats entgegen der Einführrichtung aus einem vom Haltekörper radial begrenzten Raum freigegeben wird; Abschließen der Implantation.

Gemäß eines alternativen Verfahrens weist diese zumindest die folgenden Schritte auf: Bewegen zumindest des distalen Endes des Implantats in Einführrichtung, so dass zumindest ein Bereich eines Halteelements des distalen Endes des Implantats in Einführrichtung aus einem vom Haltekörper radial begrenzten Raum freigegeben wird; Abschließen der Implantation.

Durch die erfindungsgemäßen Ausgestaltungen kann jeweils ein Verfahren realisiert werden, mittels dem eine Freigabe des Implantats von der jeweiligen Einführvorrichtung bedienerfreundlich, zuverlässig und schnell erfolgen werden kann. Bei einem so freigegebenen Implantat kann eine unerwünschte Bewegung des Implantats, z.B. Jumping, bei der Freigabe des Implantats verhindert werden. Ferner kann ein Risiko einer Deformation des Implantats sowie einer daraus resultierende Blockade der Freigabevorrichtung eliminiert werden, wodurch die Freigabe des Implantats zuverlässig und schnell erfolgen kann. Die Freigabe des Implantats gemäß der alternativen Ausgestaltung mit dem proximalen Ende zuerst ist besonders geeignet für asymmetrische Implantate, die beispielsweise bei Herzkatheteranwendungen eingesetzt werden.

Bevorzugt wird das Bewegen zumindest des distalen Endes des Implantats und damit des Bereichs eines Halteelements in Einführrichtung, vermittelt durch eine Bewegung eines der Einführelemente, insbesondere des äußeren Einführelements, in Einführrichtung oder die Bewegung zumindest des proximalen Endes des Implantats und damit des Bereichs eines Halteelements entgegen der Einführrichtung, wird vermittelt durch eine Bewegung eines der Einführelemente, insbesondere des äußeren Einführelements, entgegen der Einführrichtung. Das Bewegen zumindest des distalen Endes des Implantats in Einführrichtung oder des proximalen Endes des Implantats entgegen der Einführrichtung erfolgt bis das distale bzw. proximale Ende des Implantats an dem Stopper anschlägt. Die Expansion des Implantats kann auf zweierlei Art erfolgen. Zum einen indem das Implantat mit dem äußeren Einführelement so weit in Einführrichtung oder entgegen der Einführrichtung bewegt wird, dass das zumindest eine Halteelement aus dem zumindest einen Raum freigegeben wird bzw. austritt, woraufhin das Implantat expandiert wird oder sich selbsttätig expandiert. Und zum anderen indem das zumindest eine Halteelement nach der Bewegung des äußeren Einführelement in Einführrichtung oder entgegen der Einführrichtung in dem zumindest einen Raum verbleibt und durch eine Bewegung des inneren Einführelements um einige Millimeter, wie beispielsweise ca. 5 mm, entgegen der Einführrichtung oder in Einführrichtung freigegeben wird, woraufhin das Implantat expandiert wird oder sich selbsttätig expandiert.

Die Erfindung ist nachfolgend beispielhaft, anhand von in Zeichnungen dargestellten Ausführungsbeispielen, näher erläutert. Es zeigen in schematischer Darstellung:
- Fig. 1A: einen Schnitt durch ein günstiges Ausführungsbeispiel einer Einführvorrichtung und einer Freigabevorrichtung mit einem erfindungsgemäßen Implantat;
- Fig. 1B: eine Detaildarstellung eines Halteelements des Implantats aus Fig. 1A;
- Fig. 2: ein Haltekörper der Freigabevorrichtung der Fig. 1A und ein Stopper der Einführvorrichtung in einer Detaildarstellung;
- Fig. 3: das Implantat der Fig. 1A in einer Detaildarstellung.
- Fig. 4: die Einführvorrichtung und der Haltekörper aus Fig. 1A mit teilweise platziertem Implantat vor einem Aufschieben des äußeren Einführelements;
- Fig. 5: die Einführvorrichtung und der Haltekörper aus Fig. 1A mit vollständig platziertem Implantat nach einem Aufschieben des äußeren Einführelements;
- Fig. 6: der Haltekörper der Fig. 5 mit dem damit verbundenen Implantat in einer Detaildarstellung;
- Fig. 7: die Einführvorrichtung und der Haltekörper aus Fig. 1A mit vollständig freigegebenem Implantat;
- Fig. 8 A-F: einen Schnitt durch den Haltekörper der Fig. 1A entlang der Linie VII-VII (A) und fünf Darstellungen durch vier alternative Haltekörper analog geschnitten wie für A gezeigt (B, E, F) sowie analog geschnitten wie in Fig. 1A gezeigt (C, D);
- Fig. 9: eine alternative Einführvorrichtung und ein Haltekörper mit teilweise platziertem Implantat vor einem Aufschieben eines äußeren Einführelements;
- Fig. 10: die Einführvorrichtung und der Haltekörper aus Fig. 9 mit vollständig platziertem Implantat nach einem Aufschieben des äußeren Einführelements und
- Fig. 11: die Einführvorrichtung und der Haltekörper aus Fig. 9 mit teilweise freigegebenem Implantat.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

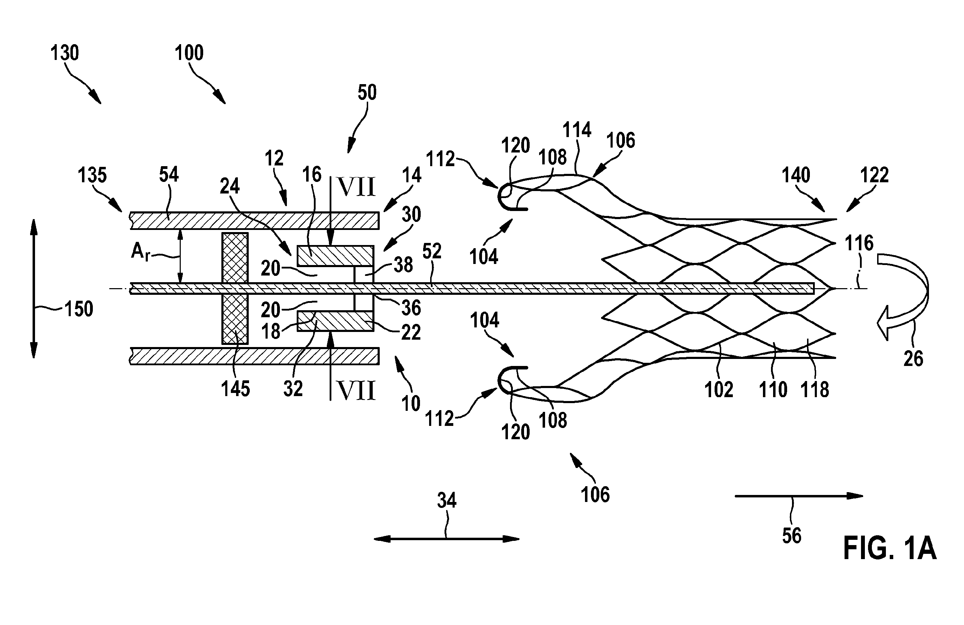

Fig. 1A zeigt einen Längsschnitt durch ein günstiges Ausführungsbeispiel einer Freigabevorrichtung 100 einer nur teilweise dargestellten Einführvorrichtung 130. Die Einführvorrichtung 130 ist beispielsweise ein Katheter mit einem Schaftbereich 50 mit zwei koaxial angeordneten Einführelementen 52, 54, z.B. einem Innenschaft (Einführelement 52) und einem diesen umgebenden Außenschaft (Einführelement 54), welcher wiederum von einer nicht gezeigten Außenhülle umgeben sein kann. Die Einführvorrichtung 130 ist in Anwenderbetrieb, also während einer Befestigung des Implantats 102 an der Freigabevorrichtung 100 oder während der Implantation mit ihrem proximalen Ende 135 einem Anwender zugewandt. Das Implantat 102 ist am distalen Ende 140 des Schaftbereichs 50 zwischen Innenschaft und Außenschaft platziert und soll am Implantationsort im tierischen oder menschlichen Körper freigegeben werden.

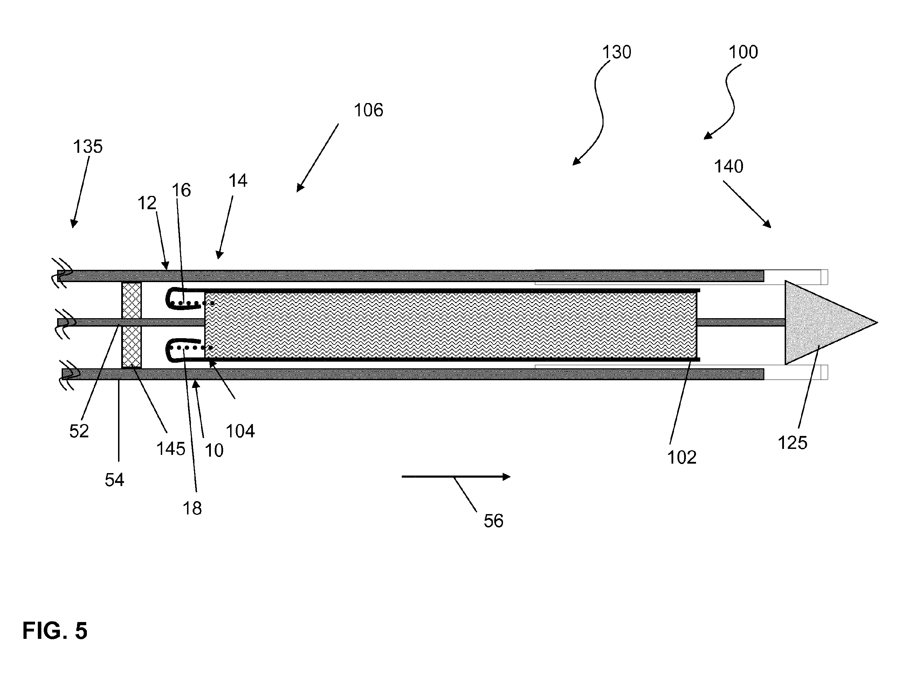

Die Freigabevorrichtung 100 dient zum Lösen des medizinischen Implantats 102 von der Einführvorrichtung 130. Das Implantat 102 ist an einem vom Anwender abgewandten Ende 140 des Schaftbereichs 50 beispielsweise in der Nähe einer Katheterspitze 125 angeordnet (siehe Fig. 5). Das Implantat 102 ist beispielsweise um das innere Einführelement 52 gelegt und wird durch eine Relativbewegung zwischen dem ersten und dem zweiten Einführelement 52, 54 in eine Einführrichtung 56 beginnend an einem distalen Ende 122 des Implantats 102 freigesetzt. Hierbei ist das innere Einführelement 52 mit der Katheterspitze 125 verbunden, das äußere Einführelement 54 hingegen nicht.

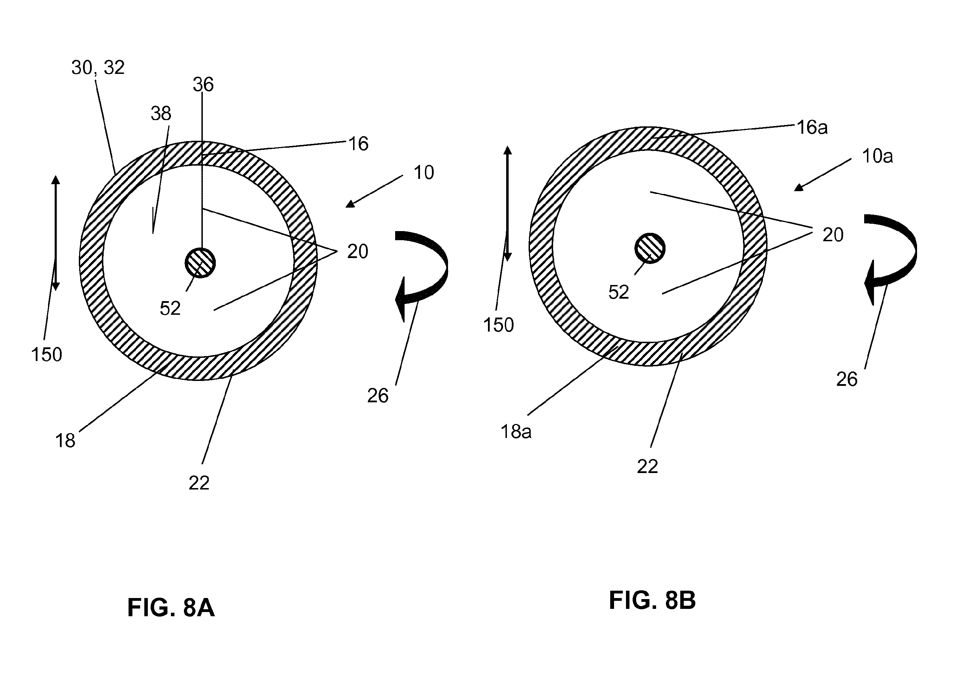

Die Freigabevorrichtung 100 umfassend einen Haltekörper 10 zum Halten des Implantats 102 in der Einführvorrichtung 130. Zudem weist der Haltekörper 10 ein proximales Ende 12, das im Benutzungszustand von dem distalen Ende 140 der Einführvorrichtung 130 entfernt ist, und ein distales Ende 14, das im Benutzungszustand dem distalen Ende 140 der Einführvorrichtung 130 zugewandt ist, auf. Der Haltekörper 10 ist von einer Buchse 30 gebildet, deren Zylindermantel 32 sich in Umfangsrichtung 26 um das innere Einführelement 52 und in axialer Richtung 34 parallel zu diesem erstreckt (siehe auch Fig. 8A). Ferner weist der Haltekörper 10 eine Durchführung 36 für das innere Einführelement 52 auf.

Der Haltekörper 10 ist mit seinem distalen Ende 14 an dem inneren Einführelemente 52 befestigt bzw. geklebt und zwar in axialer Richtung 34 zwischen einem Stopper 145 und dem distalen Ende 140 bzw. der Katheterspitze 125 der Einführvorrichtung 130. Dies erfolgt beispielsweise mittels eines UV-aushärtbaren Klebstoffs. Dieser Klebstoff bildet einen Boden 38 der Buchse 30. Der Zylindermantel 32 ist aus einem röntgensichtbaren Metall, wie beispielsweise Tantal, Gold oder Platin gebildet. Somit kann anhand eines hier nicht gezeigten Röntgengeräts während der Implantation des Implantats 102 mittels der Einführvorrichtung 130 ein Fortschritt des inneren Einführelements 52 und damit des Implantats 102 in Einführrichtung 56 sowie eine korrekte Position des Implantats 102 an einem Implantationsort überwacht werden.

Zu einer Interaktion mit dem Implantat 102 weist der Haltekörper 10 zwei Anteil 16, 18 auf, die in Umfangsrichtung 26 gleichmäßig verteilt angeordnet sind. Die zwei Anteile 16, 18 sind einstückig mit dem Zylindermantel 32 der Buchse 30 ausgeführt und dadurch an demselben Bauteil 22 ausgebildet und zwar in einer Region 24 des Bauteils 22, die sich in Umfangsrichtung 26 um das innere Einführelement 52 erstreckt. Zudem begrenzen die Anteile 16, 18 jeweils einen Raum 20 radial, wodurch sich der Raum 20 in einer radialen Richtung 150 jeweils zwischen dem inneren Einführelement 52 und dem jeweiligen Anteil 16, 18 erstreckt. Ferner ist diese Raum 20 jeweils an dem proximalen Ende 12 des Haltekörpers 10 angeordnet und stellt einen am proximalen Ende 12 des Haltekörpers 10 offenen Hohlraum dar.

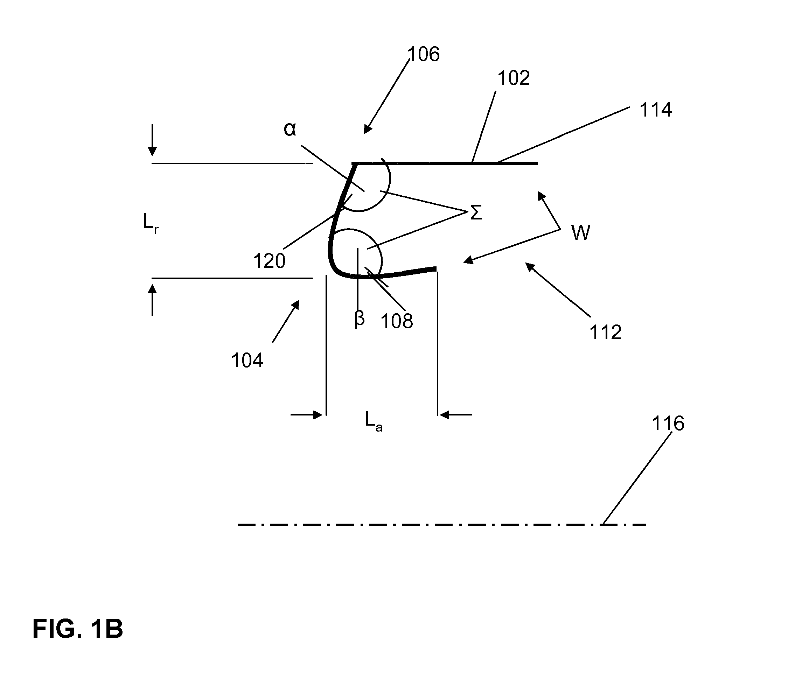

Für die Interaktion mit dem Haltekörper 10 weist das Implantat 102 zwei Halteelemente 104 auf, die gleichmäßig über einen Umfang des Implantats 102 verteilt angeordnet sind. Die Halteelemente 104 umfassen jeweils zwei Bereiche 108 und 120 und sind als Haken ausgebildet. In der Fig. 1B ist das Halteelement 104 im Detail gezeigt. Der Bereich 120 ist an einem proximalen Ende 106 des Implantats 102 angeordnet bzw. angeformt. Zudem erstreckt sich der Bereich 120 im Wesentlichen in radialer Richtung 150 zu einer Innenachse 116 des Implantats 102. Somit ist der Bereich 120 relativ zu einem Bereich 114 des Implantats 102, der sich im Wesentlichen parallel zu der Innenachse 116 des Implantats 102 erstreckt, mit einem Winkel α von ca. 90° angeordnet. Der Bereich 108 ist an einem radialen Ende des Bereichs 120 angeordnet bzw. mit diesem verbunden und schließt mit diesem einen Winkel β von ca. 90° ein. Somit erstreckt sich der Bereich 108 im Wesentlichen parallel zu der Innenachse 116 des Implantats 102 und dem Bereich 114. Durch die winklige Anordnung der Bereiche 108, 114 und 120 umfasst das Halteelement 104 relativ zu dem Bereich 114 eine Richtungsänderung 112, die einen Gesamtwinkel Σ von mehr als 90° bzw. ca. 180° aufweist.

Die Anteile 16, 18 und der Raum 20 des Haltekörpers 10 sowie die Bereiche 108 und 120 des Implantats 102 sind so aufeinander abgestimmt, dass bei der Interaktion des Halteelements 104 mit dem Haltekörper 10 der Bereich 108 des Halteelements 104 in Einführrichtung 56 in den Raum 20 einführbar und/oder entgegen der Einführrichtung 56 aus dem Raum 20 ausführbar ist.

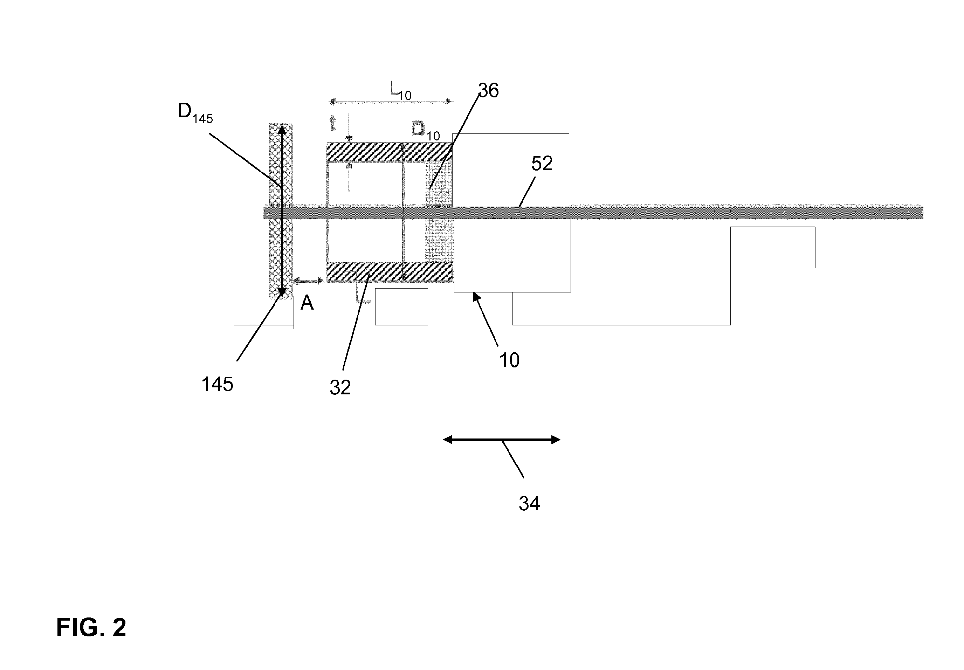

Fig. 2 zeigt den Haltekörper 10 und den Stopper 145 in einer Detaildarstellung. Die im Folgenden angegebenen Dimensionen sind ausgelegt auf ein 18F Design. Ein Außendurchmesser D₁₀ des Haltekörpers 10 beträgt beispielsweise 4 mm. Da ein radialer Abstand Aᵣ zwischen dem Außenschaft und dem Innenschaft ca. 2,1 mm beträgt, ist genügend Platz vorhanden, um den Haltekörper 10 in der Einführvorrichtung 130 platziert zu können (vgl. Fig. 1A). Eine Materialstärke t des Haltekörpers 10 ist materialabhängig und beträgt bspw. 0,2 mm. Eine axiale Länge L₁₀ des Haltekörpers 10 beträgt z. B. 5 mm. Der beispielsweise aus Polyamid gefertigte Stopper 145 hat einen Außendurchmesser D₁₄₅ von z. B. 5,3 mm. Ein axialer Abstand A zwischen dem Stopper 145 und dem Haltekörper 10 beträgt bspw. 2 mm. Eine Wandstärke W des Implantats 102 bzw. des Halteelements 104 beträgt beispielsweise 0,5 mm (siehe Fig. 1B). Eine axiale Länge Lₐ des Halteelements 104 bzw. des Bereichs 108 beträgt z. B. 1 mm und eine radiale Länge Lᵣ des Halteelements 104 bzw. des Bereichs 120 ist mehr als einmal so lang wie die Materialstärke t des Anteils 16, 18 des Haltekörpers 10 und beträgt z. B. zweimal die Materialstärke t von 0,2 mm, also bspw. 0,4 mm. Die Dimensionen in den Fig. 1B und 2 sind nicht maßstabsgetreu wiedergegeben.

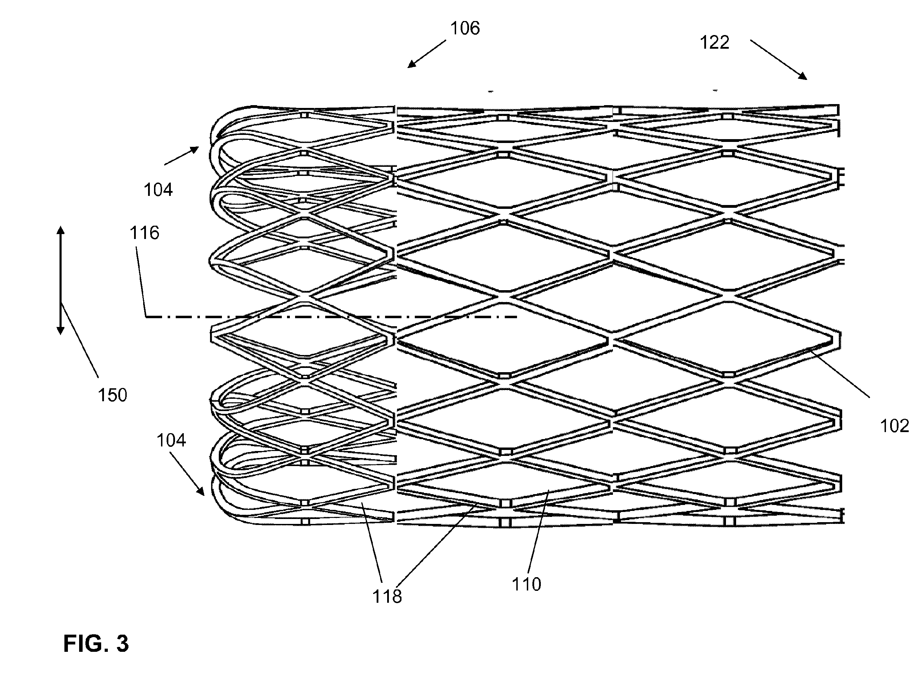

Fig. 3 zeigt das proximale Ende 106 des Implantats 102 in einer Detaildarstellung. Das Implantat 102, beispielsweise ein Stent oder ein künstliches Herzklappenimplantat, ist selbstexpandierend ausgeführt. Zudem umfasst das Implantat 102 einen Grundkörper 110, der eine Basisstruktur 118 in der Form eines Drahtgeflechts aufweist. Auch die Halteelemente 104 weisen zu mehr als 50% und zwar zu 100% diese Basisstruktur 118 auf. Gemäß eines Verfahren zur Herstellung des Implantats 102 wird das proximale Endes 106 des Implantats102 in radialer Richtung 150 zu der Innenachse 116 des Implantats 102 gebogen. Dadurch werden die Halteelemente 104 erhalten. Grundsätzlich könnte auch nur ein Anteil des proximalen Endes 106 bzw. das proximale Ende 106 nur teilweise nach innen gebogen werden.

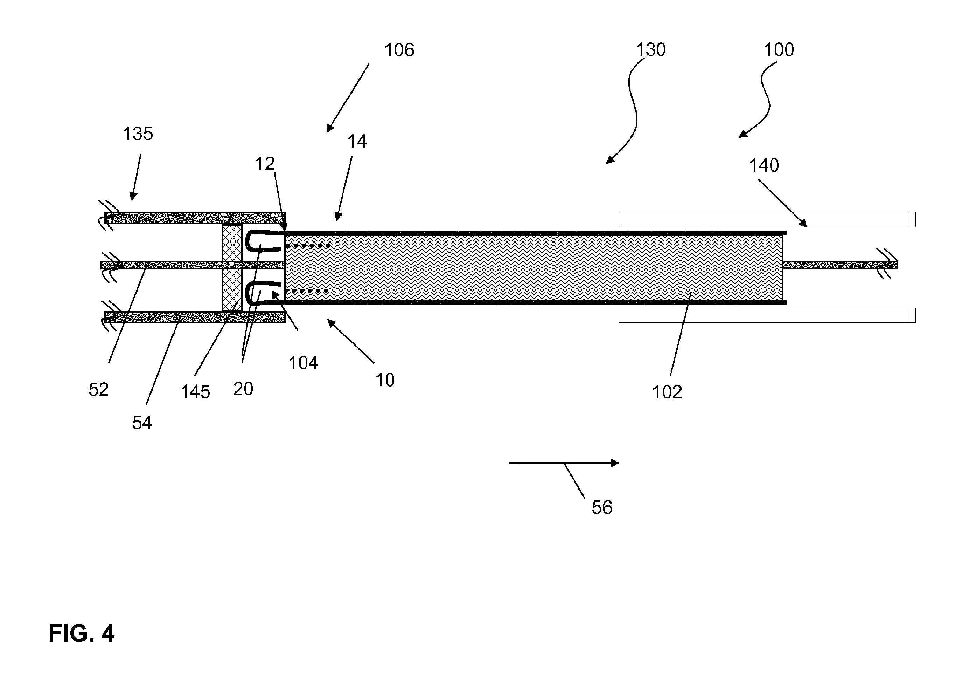

Anhand der Fig. 1A und 4 bis 6 wird nun ein Verfahren zum Verbunden des Implantats 102 mittels dem Haltekörper 10 und damit der Einführvorrichtung 130 beschrieben. In einem ersten Schritt wird das zuvor abgekühlte gecrimpte Implantat 102 entgegen der Einführrichtung 56 an/auf dem inneren Einführelemente 52 platziert. Hierbei wird das gesamte Implantat 102 über das innere Einführelement 52 und das proximale Ende 106 des Implantats 102 mit den Halteelementen 104 über den Haltekörper 10 geschoben, so dass zumindest das proximale Ende 106 des Implantats 102 proximal vom Haltekörper 10 angeordnet ist. Die Schiebebewegung erfolgt beispielsweise solange, bis das proximale Ende 106 des Implantats 102 bzw. die Bereiche 120 der Halteelemente 104 an dem Stopper 145 anschlagen (siehe Fig. 4). Der Stopper 145 limitiert somit die Bewegung des Implantats 102 in Richtung des proximalen Endes 135 der Einführvorrichtung 130. Es muss hier natürlich im Vorfeld darauf geachtet werden, dass der Abstand A zwischen dem Stopper 145 und dem Haltekörper 10 mindestens minimal breiter ist als die jeweilige axiale Länge Lₐ der Halteelements 104 bzw. des Bereichs 108.

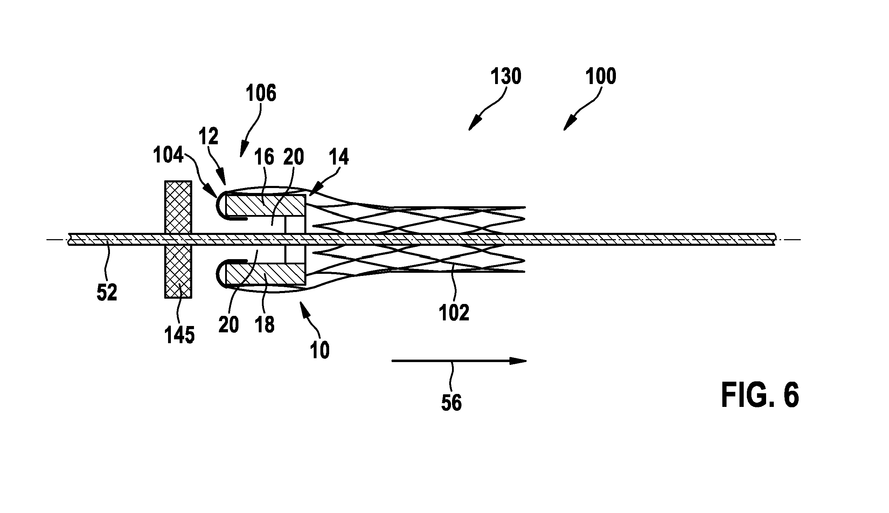

In einem zweiten Schritt wird das äußere Einführelement 54 in Einführrichtung 56 aufgeschoben, wodurch das Implantat 102 durch Haftreibung zwischen dem Einführelement 54 und dem Implantat 102 ebenso in Einführrichtung 56 bewegt wird. Hierdurch wird jeweils der Bereich 108 der Halteelemente 104 des proximalen Endes 106 des Implantats 102 in Einführrichtung 56 in den vom Haltekörper 10 radial begrenzten Raum 20 eingeführt (siehe Fig. 5 und 6). Dadurch ist der Haltekörper 10 mit dem Implantat 102 in dem äußeren Einführelement 54 platziert und die Montage ist abgeschlossen.

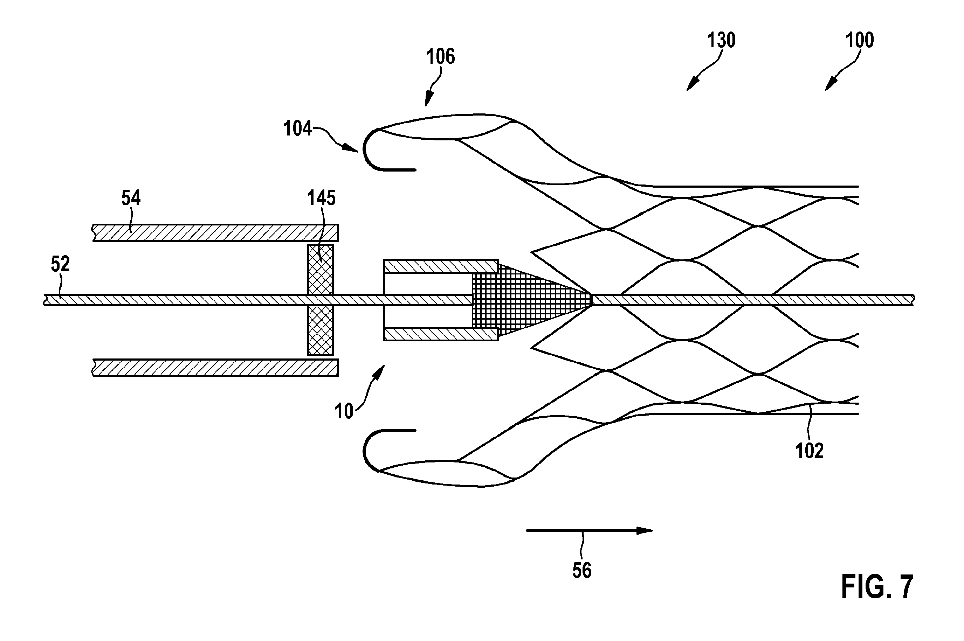

Zu einer Implantation des Implantats 102 im Körper, wird die so vorbereitete Einführvorrichtung 130 in den Körper eingeführt. Durch die Bewegung des äußeren Einführelements 54 entgegen der Einführrichtung 56 wird das distale Ende 122 des Implantats 102 freigelegt, welches sich wegen seiner Fähigkeit der Selbstexpansion öffnet und positioniert. In diesem Stadium ist das Implantat 102 noch sicher über sein proximales Ende 106 mit dem Haltekörper 10 verbunden. Es können nun bei Bedarf Funktionstests des Implantats 102 durchgeführt werden. Bei einer festgestellten Missfunktion kann das Implantat 102 wieder aus dem Körper entfernt werden. Auch eine Repositionierung eines inkorrekt positionierten Implantats 102 wäre möglich. Bei der Bewegung des äußeren Einführelements 54 wurde auch das Implantat 102 bzw. das proximale Ende 106 des Implantats 102 mit den Bereichen 108 entgegen der Einführrichtung 56 bewegt, wodurch diese aus dem vom Haltekörper 10 radial begrenzten Raum 20 freigegeben werden. Das Bewegen erfolgt beispielsweise bis das proximale Ende 106 an dem Stopper 145 anschlägt. Dieses Anstoßen an den Stopper 145 bewirkt ein Ausstoßen des Implantats 102. Hierdurch wird auch das proximale Ende 106 des Implantats 102 aufgrund seiner Radialkraft geöffnet, diese Situation ist in Fig. 7 gezeigt. Im Anschluss wird die Freigabevorrichtung 100 bzw. der Halekörper 10 mit dem Innenschaft in den Außenschaft zurückgezogen und die Einführvorrichtung 130 aus dem Körper entfernt. Das Implantat 102 verbleibt vollständig positioniert im Körper (nicht gezeigt).

Alternativ wäre auch eine zweite Art der Freisetzung des Implantats 102 möglich. Hierbei verbleiben die Bereiche 108 der Halteelemente 104 nach der Bewegung des äußeren Einführelements 54 entgegen der Einführrichtung 56 in jeweils dem Raum 20. Ein Freigabe wird hier erreicht, indem des inneren Einführelements 52 in Einführrichtung 56 ca. 5 mm bewegt wird, wodurch die Bereiche 108 aus den Räumen 20 freigegeben werden und sich das Implantat 102 selbsttätig expandiert.

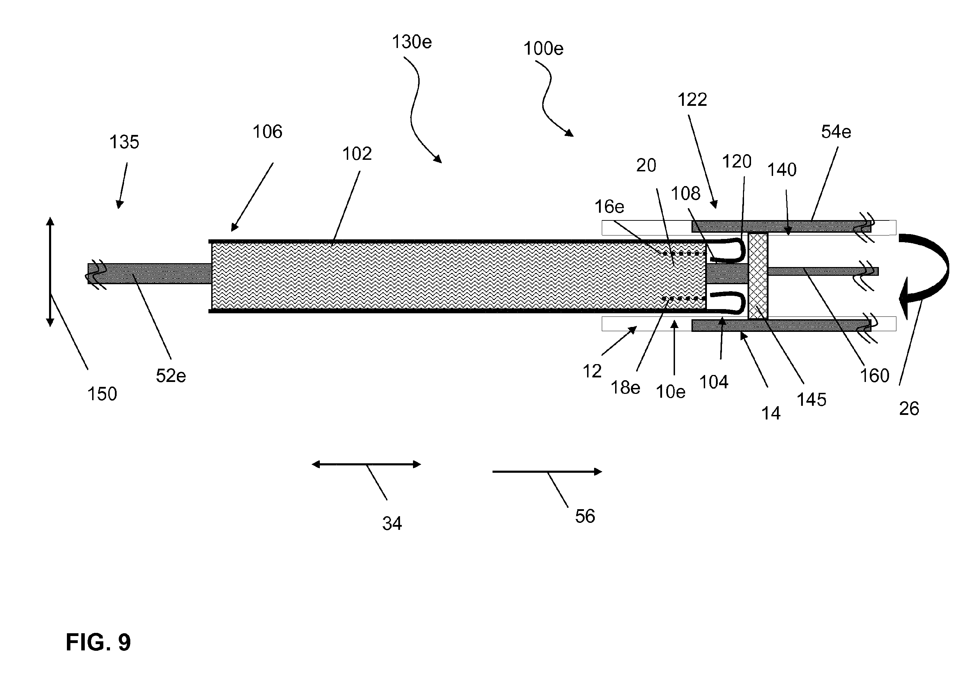

In Fig. 8B-F sind vier alternative Ausführungsbeispiele des Haltekörpers 10 und in den Fig. 9-11 ist ein alternatives Ausführungsbeispiel der Freigabevorrichtung 100 mit Haltekörper 10 und der Einführvorrichtung 130 dargestellt. Im Wesentlichen sind gleich bleibende Bauteile, Merkmale und Funktionen grundsätzlich mit den gleichen Bezugszeichen beziffert. Zur Unterscheidung des Ausführungsbeispiels der Fig. 8B-F und 9-11 zu dem in den Fig. 1A bis 8A sind jedoch den Bezugszeichen der in dem Ausführungsbeispiel der Fig. 8B-F und 9-11 abweichenden ausgeführten Bauteile der Buchstabe a bis e hinzugefügt. Die nachfolgende Beschreibung beschränkt sich im Wesentlichen auf die Unterschiede zu dem Ausführungsbeispiel in den Fig. 1A bis 8A, wobei bezüglich gleich bleibender Bauteile, Merkmale und Funktionen auf die Beschreibung des Ausführungsbeispiels in den Fig. 1A bis 8B verwiesen werden kann.

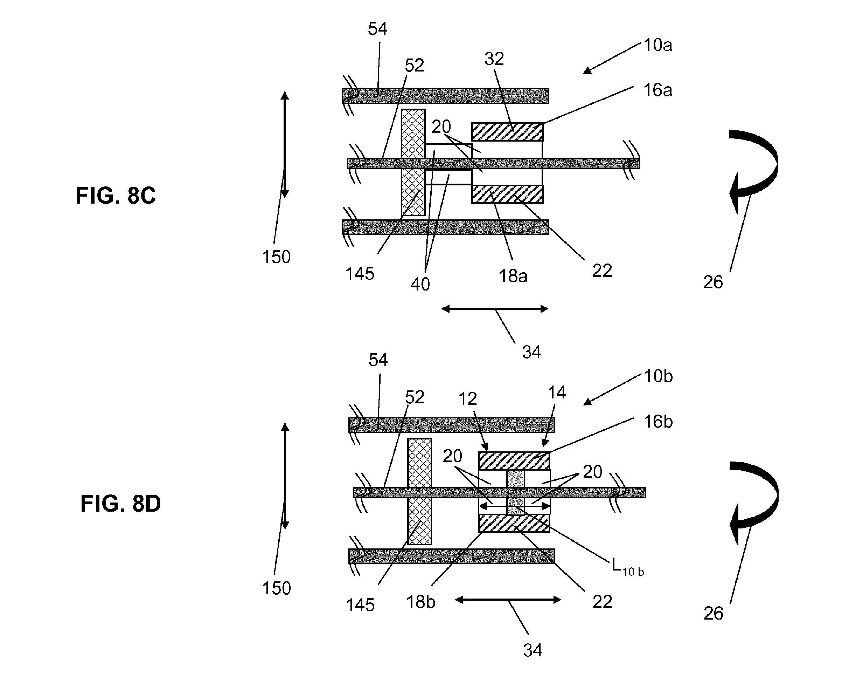

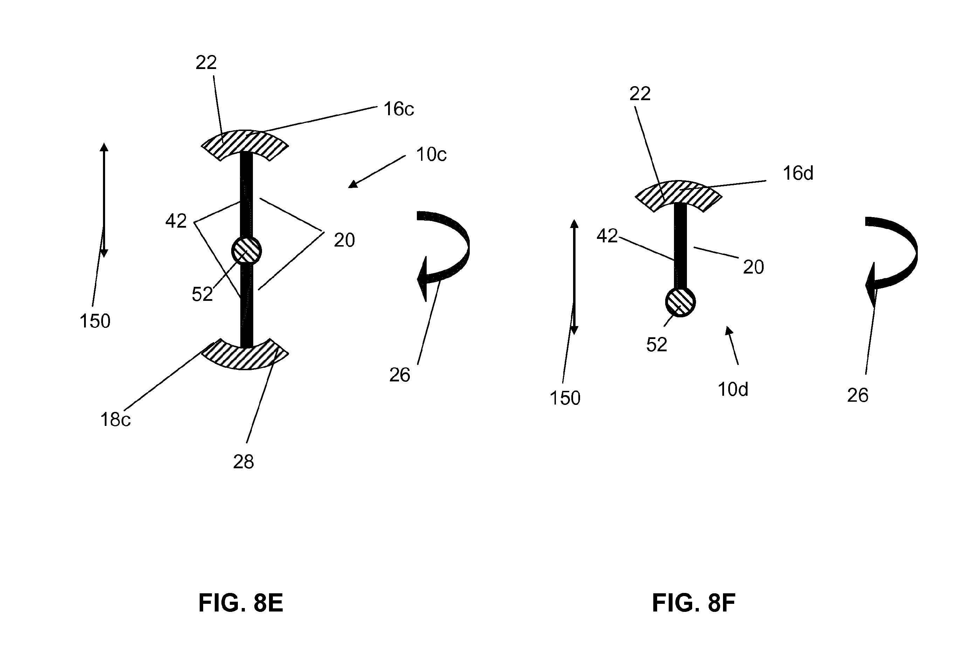

Der Haltekörper 10a der Fig. 8Bund 8C unterscheidet sich von dem Haltekörper 10 der Fig. 1A bis 8A dadurch, dass der Haltekörper 10a mittels in axialer Richtung 34 verlaufenden in Aufsicht dargestellten Stegen 40 an einem Stopper 145 befestigt bzw. an diesen geklebt ist (vgl. Fig. 8C). Um eine Interaktion von Anteilen 16a, 18a des Haltekörpers mit Halteelementen eines hier nicht gezeigten Implantats behinderungsfrei zu gestalten, sind die Stege 40 in Umfangsrichtung 26 versetzt zu den Anteilen 16a, 18a angeordnet. Ferner ist somit der Haltekörper 10 lediglich als Zylindermantel 32 ausgebildet, ein Boden entfällt, wodurch Räume 20 Hohlräume mit Öffnungen an zwei Seiten sind (vgl. Fig. 8B).

Der Haltekörper 10b der Fig. 8D unterscheidet sich von dem Haltekörper 10 der Fig. 1A bis 8A dadurch, dass der Haltekörper 10b an einem proximalen Ende 12 und an einem distalen Ende 14 Räume 20 aufweist und der Haltekörper 10b somit in axialer Richtung 34 in seiner Länge L_{10b} mittig an einem inneren Einführelement 52 befestigt ist.

Der Haltekörper 10c der Fig. 8E unterscheidet sich von dem Haltekörper 10 der Fig. 1A bis 8A dadurch, dass der Haltekörper 10c zwei Anteile 16c, 18c aufweist, die an zwei unterschiedlichen Bauteilen 22, 28 ausgebildet sind. Zur Verbindung bzw. Befestigung der Anteile 16c, 18c mit einem inneren Einführelement 52 weist der Haltekörper 10c in radialer Richtung 150 verlaufende Balken 42 auf.

Der Haltekörper 10d der Fig. 8F unterscheidet sich von dem Haltekörper 10 der Fig. 1A bis 8A dadurch, dass der Haltekörper 10d ein Anteile 16d aufweist, der mittels einem in radialer Richtung 150 verlaufenden Balken 42 mit einem inneren Einführelement 52 verbunden ist.

Fig. 9 zeigt einen Längsschnitt durch ein alternatives Ausführungsbeispiel einer Freigabevorrichtung 100e einer nur teilweise dargestellten Einführvorrichtung 130e. Die Einführvorrichtung 130e ist beispielsweise ein Katheter mit einem Schaftbereich mit zwei koaxial angeordneten Einführelementen 52e, 54e, z.B. einem Innenschaft (Einführelement 52e) und einem diesen umgebenden Außenschaft (Einführelement 54e), welcher wiederum von einer nicht gezeigten Außenhülle umgeben sein kann. Die Einführvorrichtung 130e ist in Anwenderbetrieb, also während einer Befestigung des Implantats 102 an der Freigabevorrichtung 100e oder während der Implantation mit ihrem proximalen Ende 135 einem Anwender zugewandt. Das Implantat 102 ist am distalen Ende 140 des Schaftbereichs zwischen Innenschaft und Außenschaft platziert und soll am Implantationsort im tierischen oder menschlichen Körper freigegeben werden. Das Implantat 102 ist beispielsweise um das innere Einführelement 52e gelegt und wird durch eine Relativbewegung zwischen dem ersten und dem zweiten Einführelement 52e, 54e in eine Einführrichtung 56 beginnend an einem proximalen Ende 106 des Implantats 102 freigesetzt. Hierbei ist das äußere Einführelement 54e mit der Katheterspitze 125 verbunden, das innere Einführelement 52e hingegen nicht. Zu einer Verbindung des proximalen Ende 135 des Einführvorrichtung 130e und der Katheterspitze 125 weist die Führungsvorrichtung 130e ein Führungselement 160 auf, das koaxial zu dem inneren Einführelement 52e und in diesem verläuft und beispielsweise von einem Schaft mit einem Einführungsdraht und einem Lumen gebildet ist.

Die Freigabevorrichtung 100e umfassend einen Haltekörper 10e zum Halten des Implantats 102 in der Einführvorrichtung 130e. Der Haltekörper 10e weist ein proximales Ende 12, das im Benutzungszustand von dem distalen Ende 140 der Einführvorrichtung 130e entfernt ist, und ein distales Ende 14, das im Benutzungszustand dem distalen Ende 140 der Einführvorrichtung 130e zugewandt ist, auf. Der Haltekörper 10e ist von einer Buchse 30 gebildet, deren Zylindermantel 32 sich in Umfangsrichtung 26 um das innere Einführelement 52e und in axialer Richtung 34 parallel zu diesem erstreckt (analog zu Fig. 8A). Der Haltekörper 10e ist mit seinem proximalen Ende 12 an dem inneren Einführelemente 52e befestigt bzw. geklebt und zwar in axialer Richtung 34 zwischen einem Stopper 145 und dem proximalen Ende 135 der Einführvorrichtung 130e.

Zu einer Interaktion mit dem Implantat 102 weist der Haltekörper 10e zwei Anteil 16e, 18e auf, die in Umfangsrichtung 26 gleichmäßig verteilt angeordnet sind. Die Anteile 16e, 18e begrenzen jeweils einen Raum 20 radial, wodurch sich der Raum 20 in einer radialen Richtung 150 jeweils zwischen dem inneren Einführelement 52e und dem jeweiligen Anteil 16e, 18e erstreckt. Ferner ist diese Raum 20 jeweils an dem distalen Ende 14 des Haltekörpers 10e angeordnet und stellt einen am distalen Ende 14 des Haltekörpers 10e offenen Hohlraum dar (nicht im Detail gezeigt, analog zu Fig. 1A).

Für die Interaktion mit dem Haltekörper 10e weist das Implantat 102 zwei Halteelemente 104 auf, die jeweils zwei Bereiche 108 und 120, die einen Haken bilden, aufweisen. Die Anteile 16e, 18e und der Raum 20 des Haltekörpers 10e sowie die Bereiche 108 und 120 des Implantats 102 sind so aufeinander abgestimmt, dass bei der Interaktion des Halteelements 104 mit dem Haltekörper 10e der Bereich 108 des Halteelements 104 entgegen der Einführrichtung 56 in den Raum 20 einführbar und/oder in Einführrichtung 56 aus dem Raum 20 ausführbar ist. Das Implantat 102 ist analog zu dem Implantat 102 des Ausführungsbeispiels der Fig. 1A bis 8A ausgeführt bzw. wird analog dazu hergestellt, wobei die Halteelemente 104 am distalen Ende 122 ausgebildet sind (vgl. auch Fig. 3).

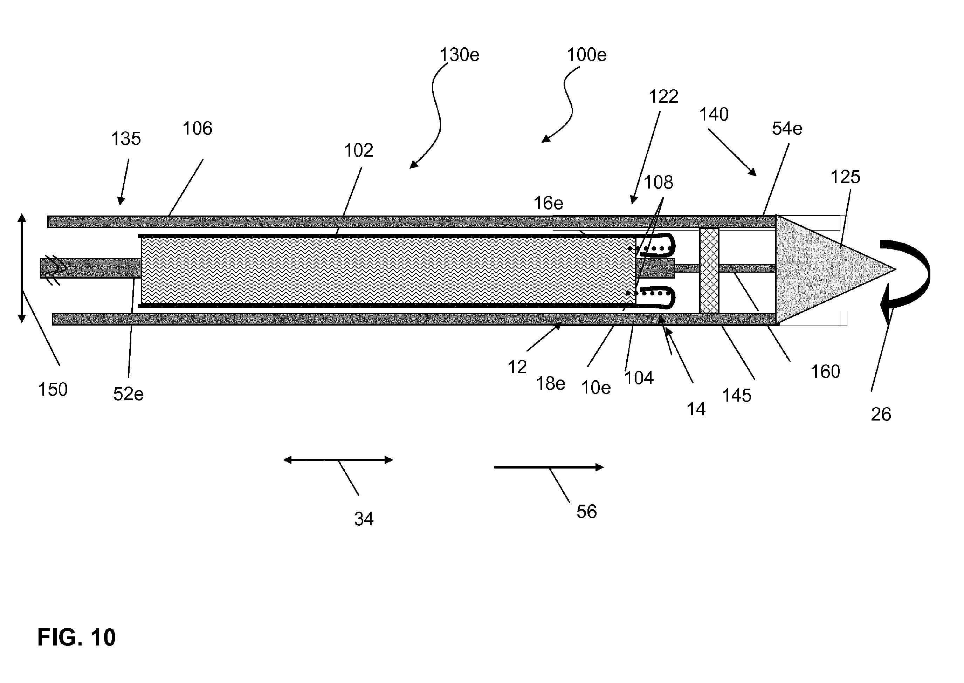

Anhand der Fig. 9 und 10 wird nun ein Verfahren zum Verbunden des Implantats 102 mittels dem Haltekörper 10e und damit der Einführvorrichtung 130e beschrieben. In einem ersten Schritt wird das zuvor abgekühlte gecrimpte Implantat 102 in Einführrichtung 56 an/auf dem inneren Einführelemente 52e platziert. Hierbei wird das gesamte Implantat 102 über das innere Einführelement 52e und das distale Ende 122 des Implantats 102 mit den Halteelementen 104 über den Haltekörper 10e geschoben, so dass zumindest das distale Ende 122 des Implantats 102 distal vom Haltekörper 10e angeordnet ist. Die Schiebebewegung erfolgt beispielsweise solange, bis das distale Ende 122 des Implantats 102 bzw. die Bereiche 120 der Halteelemente 104 an dem Stopper 145 anschlagen (siehe Fig. 9). Der Stopper 145 limitiert somit die Bewegung des Implantats 102 in Richtung des distalen Endes 140 der Einführvorrichtung 130e.

Alternativ wäre es auch möglich, dass Implantat 102 entgegen der Einführrichtung 56 auf dem Einführelement 52e zu platzieren. Dabei wird das gesamte Implantat 102 über das innere Einführelement 52e und den Stopper 145 geschoben, wobei zumindest das distale Ende 122 mit den Halteelementen 104 des Implantats 102 distal vom Haltekörper 10e angeordnet ist.

In einem zweiten Schritt wird das äußere Einführelement 54e entgegen der Einführrichtung 56 aufgeschoben, wodurch das Implantat 102 durch Haftreibung zwischen dem Einführelement 54e und dem Implantat 102 ebenso entgegen der Einführrichtung 56e bewegt wird. Hierdurch wird jeweils der Bereich 108 der Halteelemente 104 des distalen Endes 122 des Implantats 102 in Einführrichtung 56 in den vom Haltekörper 10e radial begrenzten Raum 20 eingeführt (siehe Fig. 10). Dadurch ist der Haltekörper 10e mit dem Implantat 102 in dem äußeren Einführelement 54e platziert und die Montage ist abgeschlossen.

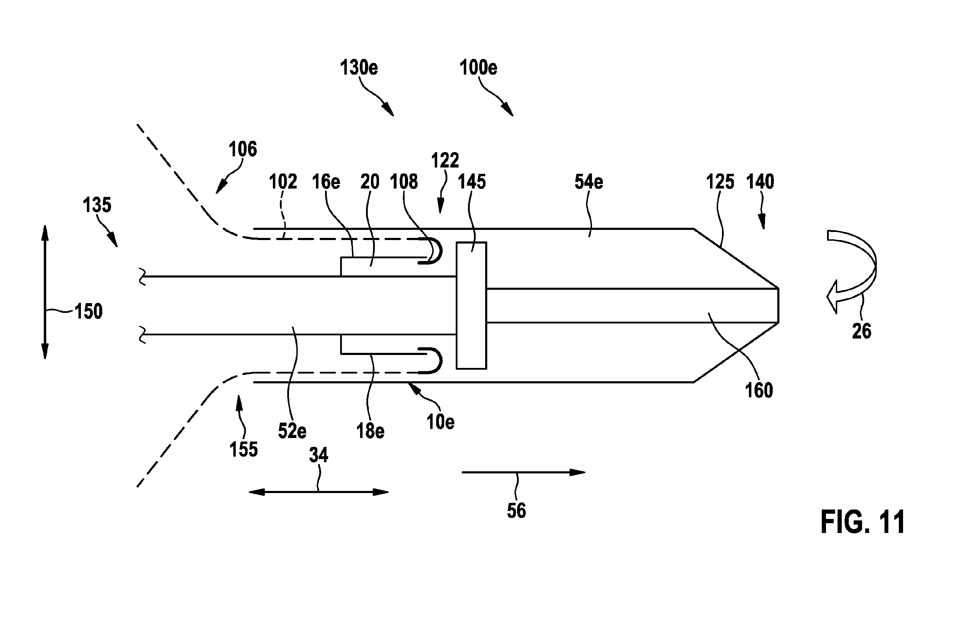

Zu einer Implantation des Implantats 102 im Körper, wird die so vorbereitete Einführvorrichtung 130e in den Körper eingeführt. Durch die Bewegung der Katheterspitze 125 und des äußeren Einführelements 54e mittels des Führungselements 160 in Einführrichtung 56 wird am äußeren Einführelement 54e eine Öffnung 155 freigelegt, die sich in Umfangsrichtung 26 erstreckt, so dass ein proximles Ende 106 des Implantats 102 freigelegt wird, welches durch die Öffnung 155 aus dem äußeren Einführelement 54e austreten kann. Hierdurch öffnet und positioniert sich das proximale Ende 106 des Implantats 102 (vgl. Fig. 11). In diesem Stadium ist das Implantat 102 noch sicher über sein distales Ende 122 mit dem Haltekörper 10e verbunden. Es können nun bei Bedarf Funktionstests des Implantats 102, eine Entfernung oder eine Repositionierung des Implantats 102 durchgeführt werden. Die Verbindung zwischen dem proximalen Ende 135 des Einführvorrichtung 130e und der Katheterspitze 125 wird durch das Führungselement 160 gewährleistet. Bei der Bewegung des äußeren Einführelements 54e wurde auch das Implantat 102 bzw. das distale Ende 122 des Implantats 102 mit den Bereichen 108 in Einführrichtung 56 bewegt, wodurch diese aus dem vom Haltekörper 10e radial begrenzten Raum 20 freigegeben werden. Das Bewegen erfolgt beispielsweise bis das distale Ende 122 an dem Stopper 145 anschlägt. Dieses Anstoßen an den Stopper 145 bewirkt ein Ausstoßen des Implantats 102. Hierdurch wird auch das distale Ende 122 des Implantats 102 aufgrund seiner Radialkraft geöffnet. Im Anschluss werden die Katheterspitze 125 und der damit verbundene Teil des äußeren Einführelements 54e mittels des Führungselements 160 wieder über die Freigabevorrichtung 100e bzw. den Halekörper 10e zurückgezogen, wodurch sich die Öffnung 155 wieder schließt. Jetzt kann die Einführvorrichtung 130e aus dem Körper entfernt werden. Das Implantat 102 verbleibt vollständig positioniert im Körper (nicht gezeigt).

Alternativ wäre auch eine zweite Art der Freisetzung des Implantats 102 möglich. Hierbei verbleiben die Bereiche 108 der Halteelemente 104 nach der Bewegung des äußeren Einführelements 54e in Einführrichtung 56 in jeweils dem Raum 20. Ein Freigabe wird hier erreicht, indem des inneren Einführelements 52e entgegen der Einführrichtung 56 ca. 5 mm bewegt wird, wodurch die Bereiche 108 aus den Räumen 20 freigegeben werden und sich das Implantat 102 selbsttätig expandiert.

**Bezugszeichen**

| | |
|---|---|
| 10 | Haltekörper |
| 12 | Ende |
| 14 | Ende |
| 16 | Anteil |
| 18 | Anteil |
| 20 | Raum |
| 22 | Bauteil |
| 24 | Region |
| 26 | Umfangsrichtung |
| 28 | Bauteil |
| 30 | Buchse |
| 32 | Zylindermantel |
| 34 | axiale Richtung |
| 36 | Durchführung |
| 38 | Boden |
| 40 | Steg |
| 42 | Balken |
| 50 | Schaftbereich |
| 52 | Einführelement |
| 54 | Einführelement |
| 56 | Einführrichtung |
| 100 | Freigabevorrichtung |
| 102 | Implantat |
| 104 | Halteelement |
| 106 | Ende |
| 108 | Bereich |
| 110 | Grundkörper |
| 112 | Richtungsänderung |
| 114 | Bereich |
| 116 | Innenachse |
| 118 | Basisstruktur |
| 120 | Bereich |
| 122 | Ende |
| 125 | Katheterspitze |
| 130 | Einführvorrichtung |
| 135 | Ende |
| 140 | Ende |
| 145 | Stopper |
| 150 | radiale Richtung |
| 155 | Öffnung |
| 160 | Führungselement |
| A | Abstand |
| D | Außendurchmesser |
| L | Länge |
| t | Materialstärke |
| W | Wandstärke |
| α | Winkel |
| β | Winkel |
| ∑ | Gesamtwinkel |

## Patentansprüche

1. Freigabevorrichtung (100) zum Lösen eines medizinischen Implantats (102) von einer Einführvorrichtung (130), bei welcher das Implantat (102) durch eine Relativbewegung zwischen einem ersten und einem zweiten Einführelement (52, 54) in eine Einführrichtung (56) zuerst an seinem distalen Ende (122) freisetzbar ist, umfassend einen Haltekörper (10, 10a-10d) zum Halten des Implantats (102) in der Einführvorrichtung (130), mit einem proximalen Ende (12), das im Benutzungszustand von einem distalen Ende (140) der Einführvorrichtung (130) entfernt ist, und einem distalen Ende (14), das im Benutzungszustand dem distalen Ende (140) der Einführvorrichtung (130) zugewandt ist, wobei der Haltekörper (10, 10a-10d) zumindest einen Anteil (16, 16a-16d; 18, 18a-18c) aufweist, der zumindest einen Raum (20) radial begrenzt, der sich zwischen einem der Einführelemente (52) und dem zumindest einen Anteil (16, 16a-16d; 18, 18a-18c) erstreckt, wobei das Implantat (102) zumindest ein Halteelement (104) an zumindest seinem proximalen Ende (106) zur Interaktion mit dem Haltekörper (10, 10a-10d) aufweist, und wobei zumindest ein Bereich (108) des zumindest einen Halteelements (104) bei der Interaktion mit dem Haltekörper (10, 10a-10d) in Einführrichtung (56) in den zumindest einen Raum (20) einführbar und/oder entgegen der Einführrichtung (56) aus dem zumindest einen Raum (20) ausführbar ist.

2. Freigabevorrichtung (100e) zum Lösen eines medizinischen Implantats (102) von einer Einführvorrichtung (130e), bei welcher das Implantat (102) durch eine Relativbewegung zwischen einem ersten und einem zweiten Einführelement (52e, 54e) in eine Einführrichtung (56) zuerst an seinem proximalen Ende (106) freisetzbar ist, umfassend einen Haltekörper (10a-10e) zum Halten des Implantats (102) in der Einführvorrichtung (130e), mit einem proximalen Ende (12), das im Benutzungszustand von einem distalen Ende (140) der Einführvorrichtung (130e) entfernt ist, und einem distalen Ende (14), das im Benutzungszustand dem distalen Ende (140) der Einführvorrichtung (130e) zugewandt ist, wobei der Haltekörper (10a-10e) zumindest einen Anteil (16a-16e; 18a-18c, 18e) aufweist, der zumindest einen Raum (20) radial begrenzt, der sich zwischen einem der Einführelemente (52e) und dem zumindest einen Anteil (16a-16e; 18a-18c, 18e) erstreckt, wobei das Implantat (102) zumindest ein Halteelement (104) an zumindest seinem distalen Ende (122) zur Interaktion mit dem Haltekörper (10a-10e) aufweist, und wobei zumindest ein Bereich (108) des zumindest einen Halteelements (104) bei der Interaktion mit dem Haltekörper (10a-10e) entgegen der Einführrichtung (56) in den zumindest einen Raum (20) einführbar und/oder in Einführrichtung (56) aus dem zumindest einen Raum (20) ausführbar ist.

3. Freigabevorrichtung nach Anspruch 1 oder 2, wobei der zumindest eine Raum (20) an dem proximalen Ende (12) des Haltekörpers (10, 10a-10d) oder an dem distalen Ende (14) des Haltekörpers (10a-10e) angeordnet ist.

4. Freigabevorrichtung nach einem der vorhergehenden Ansprüche, wobei der Haltekörper (10, 10a-10e) an einem der Einführelemente (52, 52e) befestigt ist, insbesondere mit seinem proximalen Ende (12) oder seinem distalen Ende (14).

5. Freigabevorrichtung nach einem der vorhergehenden Ansprüche, wobei der Haltekörper (10, 10b-10e) auf ein inneres Einführelement (52, 52e) geklebt ist, insbesondere mittels eines UV-aushärtbaren Klebstoffs.

6. Freigabevorrichtung nach einem der vorhergehenden Ansprüche, wobei der Haltekörper (10, 10a-10c, 10e) zumindest zwei Anteile (16, 16a-16c, 16e; 18, 18a-18c, 18e) aufweist, die jeweils zumindest einen Raum (20) radial begrenzen, der sich jeweils zwischen einem inneren Einführelement (52, 52e) und dem Anteil (16, 16a-16c; 18, 18a-18c) erstreckt.

7. Freigabevorrichtung nach Anspruch 6, wobei die zumindest zwei Anteile (16, 16a, 16b, 16e; 18, 18a, 18b, 18e) an demselben Bauteil (22) ausgebildet sind und/oder in einer Region (24) eines Bauteils (22) angeordnet sind, die sich in Umfangsrichtung (26) um eines der Einführelemente (52, 52e) erstreckt.

8. Freigabevorrichtung nach Anspruch 6, wobei die zumindest zwei Anteile (16c; 18c) an zwei unterschiedlichen Bauteilen (22, 28) ausgebildet sind.

9. Freigabevorrichtung nach einem der vorhergehenden Ansprüche, wobei der Haltekörper (10, 10a-10e) ein Material aufweist, das ausgewählt ist aus der Gruppe bestehend aus einer Keramik, einem Glas, einem Gummi, einem Kunststoff, einem Metall und einem röntgensichtbaren Metall und insbesondere aus Polyamid, AcrylnitrilButadien-Styrol, Stahl, Nitinol, Tantal, Gold oder Platin.

10. Einführvorrichtung (130) zum Einführen eines medizinischen Implantats (102), welches durch eine Relativbewegung zwischen einem ersten und einem zweiten Einführelement (52, 54) in eine Einführrichtung (56) zuerst an seinem distalen Ende (122) freisetzbar ist, umfassend eine Freigabevorrichtung (100) zum Lösen des medizinisches Implantats (102), insbesondere nach einem der Ansprüche 1 und 3 bis 9, umfassend einen Haltekörper (10, 10a-10d) zum Halten des Implantats (102) in der Einführvorrichtung (130), mit einem proximalen Ende (12), das im Benutzungszustand von einem distalen Ende (140) der Einführvorrichtung (130) entfernt ist, und einem distalen Ende (14), das im Benutzungszustand dem distalen Ende (140) der Einführvorrichtung (130) zugewandt ist, wobei der Haltekörper (10, 10a-10d) zumindest einen Anteil (16, 16a-16d; 18, 18a-18c) aufweist, der zumindest einen Raum (20) radial begrenzt, der sich zwischen einem der Einführelemente (52) und dem zumindest einen Anteil (16, 16a-16d; 18, 18a-18c) erstreckt, wobei das Implantat (102) zumindest ein Halteelement (104) an zumindest seinem proximalen Ende (106) zur Interaktion mit dem Haltekörper (10, 10a-10e) aufweist, und wobei zumindest ein Bereich (108) des zumindest einen Halteelements (104) bei der Interaktion mit dem Haltekörper (10, 10a-10d) in Einführrichtung (56) in den zumindest einen Raum (20) einführbar und/oder entgegen der Einführrichtung (56) aus dem zumindest einen Raum (20) ausführbar ist.

11. Einführvorrichtung (130e) zum Einführen eines medizinischen Implantats (102), welches durch eine Relativbewegung zwischen einem ersten und einem zweiten Einführelement (52e, 54e) in eine Einführrichtung (56) zuerst an seinem proximalen Ende (106) freisetzbar ist, umfassend eine Freigabevorrichtung (100e) zum Lösen des medizinisches Implantats (102), insbesondere nach einem der Ansprüche 2 bis 9, umfassend einen Haltekörper (10a-10e) zum Halten des Implantats (102) in der Einführvorrichtung (130e), mit einem proximalen Ende (12), das im Benutzungszustand von einem distalen Ende (140) der Einführvorrichtung (130e) entfernt ist, und einem distalen Ende (14), das im Benutzungszustand dem distalen Ende (140) der Einführvorrichtung (130e) zugewandt ist, wobei der Haltekörper (10a-10e) zumindest einen Anteil (16a-16e; 18a-18c, 18e) aufweist, der zumindest einen Raum (20) radial begrenzt, der sich zwischen einem der Einführelemente (52e) und dem zumindest einen Anteil (16a-16e; 18a-18c, 18e) erstreckt, wobei das Implantat (102) zumindest ein Halteelement (104) an zumindest seinem distalen Ende (122) zur Interaktion mit dem Haltekörper (10a-10e) aufweist, und wobei zumindest ein Bereich (108) des zumindest einen Halteelements (104) bei der Interaktion mit dem Haltekörper (10a-10e) entgegen der Einführrichtung (56) in den zumindest einen Raum (20) einführbar und/oder in Einführrichtung (56) aus dem zumindest einen Raum (20) ausführbar ist.

12. Einführvorrichtung nach Anspruch 10 oder 11, wobei ein Stopper (145) vorgesehen ist, der eine Bewegung des Implantats (102) in Richtung eines proximalen Endes (135) der Einführvorrichtung (130) oder in Richtung des distalen Endes (140) der Einführvorrichtung (130e) limitiert und/oder ein Ausstoßen des Implantats (102) bewirkt.

13. Medizinisches Implantat (102) mit einem Grundkörper (110) zum Verbinden mit einer Freigabevorrichtung (100), insbesondere nach einem der Ansprüche 1 und 3 bis 9, und zum Lösen von einer Einführvorrichtung (130), insbesondere nach einem der Ansprüche 10 und 12, wobei das Implantat (102) durch eine Relativbewegung zwischen einem ersten und einem zweiten Einführelement (52, 54) in eine Einführrichtung (56) zuerst an seinem distalen Ende (122) freisetzbar ist, aufweisend zumindest ein Halteelement (104) zur Interaktion mit einem Haltekörper (10, 10a-10d) der Freigabevorrichtung (100), wobei das zumindest eine Halteelement (104) zumindest eine Richtungsänderung (112) umfasst, die relativ zu einem Bereich (114) des Grundkörpers (110), der sich im Wesentlichen parallel zu einer Innenachse (116) des Grundkörpers (110) erstreckt, einen Gesamtwinkel (Σ) von mehr als 90° aufweist und in radialer Richtung (150) zu der Innenachse (116) orientiert angeordnet ist, so dass bei der Interaktion mit dem Haltekörper (10, 10a-10d) zumindest ein Bereich (108) des zumindest einen Halteelements (104) in Einführrichtung (56) in den zumindest einen Raum (20) des Haltekörpers (10, 10a-10d) einführbar und/oder entgegen der Einführrichtung (56) aus dem zumindest einen Raum (20) ausführbar ist.

14. Medizinisches Implantat (102) mit einem Grundkörper (110) zum Verbinden mit einer Freigabevorrichtung (100e), insbesondere nach einem der Ansprüche 2 bis 9, und zum Lösen von einer Einführvorrichtung (130e), insbesondere nach einem der Ansprüche 11 oder 12, wobei das Implantat (102) durch eine Relativbewegung zwischen einem ersten und einem zweiten Einführelement (52e, 54e) in eine Einführrichtung (56) zuerst an seinem proximalen Endes (106) freisetzbar ist, aufweisend zumindest ein Halteelement (104) zur Interaktion mit einem Haltekörper (10a-10e) der Freigabevorrichtung (100e), wobei das zumindest eine Halteelement (104) zumindest eine Richtungsänderung (112) umfasst, die relativ zu einem Bereich (114) des Grundkörpers (110), der sich im Wesentlichen parallel zu einer Innenachse (116) des Grundkörpers (110) erstreckt, einen Gesamtwinkel (Σ) von mehr als 90° aufweist und in radialer Richtung (150) zu der Innenachse (116) orientiert angeordnet ist, so dass bei der Interaktion mit dem Haltekörper (10a-10e) zumindest ein Bereich (108) des zumindest einen Halteelements (104) entgegen der Einführrichtung (56) in den zumindest einen Raum (20) des Haltekörpers (10a-10e) einführbar und/oder in Einführrichtung (56) aus dem zumindest einen Raum (20) ausführbar ist.

15. Medizinisches Implantat nach Anspruch 13 oder 14, wobei der Grundkörper (110) zumindest eine Basisstruktur (118) aufweist und wobei das zumindest ein Halteelement (104) zumindest zu mehr als 50% die Basisstruktur (118) des Grundkörpers (110) aufweist.

16. Medizinisches Implantat nach einem der Ansprüche 13 bis 15, wobei das zumindest eine Halteelement (104) einen Bereich (120) aufweist, der sich im Wesentlichen in radialer Richtung (150) zu der Innenachse (116) des Implantats (102) erstreckt und in seiner Länge (Lᵣ) mehr als einmal so lang ist wie eine Materialstärke (t) des Anteils (16, 16a-16e; 18, 18a-18c, 18e) des Haltekörpers (10, 10a-10e) der Freigabevorrichtung (100, 100e).

17. Medizinisches Implantat nach einem der Ansprüche 13 bis 16, wobei das Implantat (102) als ein selbstexpandierendes Implantat (102) ausgebildet ist.

18. Verfahren zum Verbinden eines medizinischen Implantats (102), insbesondere nach einem der Ansprüche 13 und 15 bis 17, mittels eines Haltekörpers (10, 10a-10d) einer Freigabevorrichtung (100), insbesondere nach einem der Ansprüche 1 und 3 bis 9, in einer Einführvorrichtung (130), insbesondere nach einem der Ansprüche 10 oder 12, bei welcher das Implantat (102) durch eine Relativbewegung zwischen einem ersten und einem zweiten Einführelement (52, 54) in eine Einführrichtung (56) zuerst an seinem distalen Ende (122) freigesetzt wird, wobei der Haltekörper (10, 10a-10d) ein proximales Ende (12), das im Benutzungszustand von einem distalen Ende (140) der Einführvorrichtung (130) entfernt ist, und ein distales Ende (14), das im Benutzungszustand dem distalen Ende (140) der Einführvorrichtung (130) zugewandt ist, umfasst, aufweisend zumindest die folgenden Schritte:
- Platzieren zumindest eines proximalen Endes (106) des Implantats (102) entgegen der Einführrichtung (56) an einem der Einführelemente (52), so dass zumindest das proximale Ende (106) des Implantats (102) proximal von dem Haltekörper (10, 10a-10d) angeordnet ist;
- Bewegen zumindest des proximalen Endes (106) des Implantats (102) in Einführrichtung (56), so dass zumindest ein Bereich (108) zumindest eines Halteelements (104) des proximalen Endes (106) des Implantats (102) in Einführrichtung (56) in einen vom Haltekörper (10, 10a-10d) radial begrenzten Raum (20) eingeführt wird;
- Platzierung des Haltekörpers (10, 10a-10d) mit dem Implantat (102) in zumindest einem der Einführelemente (54).

19. Verfahren zum Verbinden eines medizinischen Implantats (102), insbesondere nach einem der Ansprüche 14 bis 17, mittels eines Haltekörpers (10a-10e) einer Freigabevorrichtung (100e), insbesondere nach einem der Ansprüche 2 bis 9, in einer Einführvorrichtung (130e), insbesondere nach einem der Ansprüche 11 oder 12, bei welcher das Implantat (102) durch eine Relativbewegung zwischen einem ersten und einem zweiten Einführelement (52e, 54e) in eine Einführrichtung (56) zuerst an seinem proximalen Ende (106) freigesetzt wird, wobei der Haltekörper (10a-10e) ein proximales Ende (12), das im Benutzungszustand von einem distalen Ende (140) der Einführvorrichtung (130) entfernt ist, und ein distales Ende (14), das im Benutzungszustand dem distalen Ende (140) der Einführvorrichtung (130) zugewandt ist, umfasst, aufweisend zumindest die folgenden Schritte:
- Platzieren zumindest eines distalen Endes (122) des Implantats (102) in Einführrichtung (56) an einem der Einführelemente (52e), so dass zumindest das distale Ende (122) des Implantats (102) distal von dem Haltekörper (10a-10e) angeordnet ist;
- Bewegen zumindest des distalen Endes (122) des Implantats (102) entgegen der Einführrichtung (56), so dass zumindest ein Bereich (108) zumindest eines Halteelements (104) des distalen Endes (122) des Implantats (102) entgegen der Einführrichtung (56) in einen vom Haltekörper (10a-10e) radial begrenzten Raum (20) eingeführt wird;
- Platzierung des Haltekörpers (10a-10e) mit dem Implantat (102) in zumindest einem der Einführelemente (54e).
